# EUROPEAN PATENT APPLICATION

(11) **EP 3 950 708 A1**
(43) Date of publication of application: **09.02.2022**
(21) Application number: 20783077.9
(22) Date of filing: 01.04.2020
(51) Int. Cl.: C07K 14/435

(54) **METHOD FOR EXPRESSING AND PURIFYING PROTEIN BY USING CSQ-TAG**

(30) Priority: 05.04.2019 KR 20190040241; 31.03.2020 KR 20200039185
(71) Applicant: Korea Institute of Ceramic Engineering and Technology, Jinju-si, Gyeongsangnam-do 52851 (KR)
(72) Inventor: KIM, Sung Hyun, Sejong 30098 (KR); BANG, Jin Ho, Sejong 30024 (KR); CHOI, Mi Hyun, Busan 46983 (KR)
(74) Representative: Eisenführ Speiser
(86) International application number: PCT/KR2020/004463
(87) International publication number: WO 2020/204601

(57) **Abstract**

The present invention relates to a method for expressing, water-solubilizing, and purifying protein by using a calsequestrin-tag (CSQ-tag). Provided are: a recombinant expression vector containing a CSQ-tag and a target protein; a host cell transformed using the recombinant expression vector; and a method for expressing and purifying a target protein by using a CSQ tag. The present invention uses CSQ-tags to increase the expression of proteins that are widely used in pharmaceuticals and cosmetics, and allows the proteins to be easily separated by calcium, and thus is expected to be able to lower the cost of protein materials and protein pharmaceuticals.

<110>    Korea Institute of Ceramic Engineering and Technology

<120>    METHOD OF EXPRESSING AND PURIFYING PROTEIN USING CSQ-TAG

<130>    OP-2020-0010-KR

<150>    KR 10-2019-0040241
<151>    2019-04-05

<160>    25

<170>    KopatentIn 2.0

<210>    1
<211>    362
<212>    PRT
<213>    Artificial Sequence

<220>
<223>    CSQ1


<400>    1
Gln Glu Gly Leu Asp Phe Pro Glu Tyr Asp Gly Val Asp Arg Val Ile
  1               5                  10                  15

Asn Val Asn Ala Lys Asn Tyr Lys Asn Val Phe Lys Lys Tyr Glu Val
             20                  25                  30

Leu Ala Leu Leu Tyr His Glu Pro Pro Glu Asp Asp Lys Ala Ser Gln
         35                  40                  45

Arg Gln Phe Glu Met Glu Glu Leu Ile Leu Glu Leu Ala Ala Gln Val
     50                  55                  60

Leu Glu Asp Lys Gly Val Gly Phe Gly Leu Val Asp Ser Glu Lys Asp
 65                  70                  75                  80

Ala Ala Val Ala Lys Lys Leu Gly Leu Thr Glu Val Asp Ser Met Tyr
                 85                  90                  95

Val Phe Lys Gly Asp Glu Val Ile Glu Tyr Asp Gly Glu Phe Ser Ala
            100                 105                 110

Asp Thr Ile Val Glu Phe Leu Leu Asp Val Leu Glu Asp Pro Val Glu
        115                 120                 125

Leu Ile Glu Gly Glu Arg Glu Leu Gln Ala Phe Glu Asn Ile Glu Asp
    130                 135                 140

Glu Ile Lys Leu Ile Gly Tyr Phe Lys Ser Lys Asp Ser Glu His Tyr
145                 150                 155                 160

Lys Ala Phe Glu Asp Ala Ala Glu Glu Phe His Pro Tyr Ile Pro Phe
                165                 170                 175

Phe Ala Thr Phe Asp Ser Lys Val Ala Lys Lys Leu Thr Leu Lys Leu
            180                 185                 190

Asn Glu Ile Asp Phe Tyr Glu Ala Phe Met Glu Glu Pro Val Thr Ile
        195                 200                 205

Pro Asp Lys Pro Asn Ser Glu Glu Glu Ile Val Asn Phe Val Glu Glu

210                 215                 220

His Arg Arg Ser Thr Leu Arg Lys Leu Lys Pro Glu Ser Met Tyr Glu
225                 230                 235                 240

Thr Trp Glu Asp Asp Met Asp Gly Ile His Ile Val Ala Phe Ala Glu
                245                 250                 255

Glu Ala Asp Pro Asp Gly Phe Glu Phe Leu Glu Thr Leu Lys Ala Val
            260                 265                 270

Ala Gln Asp Asn Thr Glu Asn Pro Asp Leu Ser Ile Ile Trp Ile Asp
        275                 280                 285

Pro Asp Asp Phe Pro Leu Leu Val Pro Tyr Trp Glu Lys Thr Phe Asp
    290                 295                 300

Ile Asp Leu Ser Ala Pro Gln Ile Gly Val Val Asn Val Thr Asp Ala
305                 310                 315                 320

Asp Ser Val Trp Met Glu Met Asp Asp Glu Glu Asp Leu Pro Ser Ala
                325                 330                 335

Glu Glu Leu Glu Asp Trp Leu Glu Asp Val Leu Glu Gly Glu Ile Asn
            340                 345                 350

Thr Glu Asp Asp Asp Asp Asp Asp Asp Asp
        355                 360


<210>    2
<211>    380
<212>    PRT
<213>    Artificial Sequence

<220>
<223>    CSQ2


<400>    2
   Glu Glu Gly Leu Asn Phe Pro Thr Tyr Asp Gly Lys Asp Arg Val Val
    1               5                  10                  15
   Ser Leu Ser Glu Lys Asn Phe Lys Gln Val Leu Lys Lys Tyr Asp Leu
               20                  25                  30
   Leu Cys Leu Tyr Tyr His Glu Pro Val Ser Ser Asp Lys Val Thr Pro
           35                  40                  45
   Lys Gln Phe Gln Leu Lys Glu Ile Val Leu Glu Leu Val Ala Gln Val
       50                  55                  60
   Leu Glu His Lys Ala Ile Gly Phe Val Met Val Asp Ala Lys Lys Glu
   65                  70                  75                  80
   Ala Lys Leu Ala Lys Lys Leu Gly Phe Asp Glu Glu Gly Ser Leu Tyr
                   85                  90                  95
   Ile Leu Lys Gly Asp Arg Thr Ile Glu Phe Asp Gly Glu Phe Ala Ala
               100                 105                 110
   Asp Val Leu Val Glu Phe Leu Leu Asp Leu Ile Glu Asp Pro Val Glu
           115                 120                 125
   Ile Ile Ser Ser Lys Leu Glu Val Gln Ala Phe Glu Arg Ile Glu Asp
       130                 135                 140
   Tyr Ile Lys Leu Ile Gly Phe Phe Lys Ser Glu Asp Ser Glu Tyr Tyr
   145                 150                 155                 160
   Lys Ala Phe Glu Glu Ala Ala Glu His Phe Gln Pro Tyr Ile Lys Phe
                   165                 170                 175
   Phe Ala Thr Phe Asp Lys Gly Val Ala Lys Lys Leu Ser Leu Lys Met
               180                 185                 190
   Asn Glu Val Asp Phe Tyr Glu Pro Phe Met Asp Glu Pro Ile Ala Ile
           195                 200                 205
   Pro Asn Lys Pro Tyr Thr Glu Glu Glu Leu Val Glu Phe Val Lys Glu
       210                 215                 220
   His Gln Arg Pro Thr Leu Arg Arg Leu Arg Pro Glu Glu Met Phe Glu
   225                 230                 235                 240
   Thr Trp Glu Asp Asp Leu Asn Gly Ile His Ile Val Ala Phe Ala Glu
                   245                 250                 255
   Lys Ser Asp Pro Asp Gly Tyr Glu Phe Leu Glu Ile Leu Lys Gln Val
               260                 265                 270
   Ala Arg Asp Asn Thr Asp Asn Pro Asp Leu Ser Ile Leu Trp Ile Asp
           275                 280                 285
   Pro Asp Asp Phe Pro Leu Leu Val Ala Tyr Trp Glu Lys Thr Phe Lys
       290                 295                 300
   Ile Asp Leu Phe Arg Pro Gln Ile Gly Val Val Asn Val Thr Asp Ala
   305                 310                 315                 320
   Asp Ser Val Trp Met Glu Ile Pro Asp Asp Asp Asp Leu Pro Thr Ala
                   325                 330                 335
   Glu Glu Leu Glu Asp Trp Ile Glu Asp Val Leu Ser Gly Lys Ile Asn
               340                 345                 350
   Thr Glu Asp Asp Asp Glu Asp Asp Asp Asp Asp Asp Asn Ser Asp Glu
           355                 360                 365
   Glu Asp Asn Asp Asp Ser Asp Asp Asp Asp Asp Glu
       370                 375                 380


<210>    3
<211>    1086
<212>    DNA
<213>    Artificial Sequence

<220>
<223>    CSQ1


<400>    3
caggaagggc tggacttccc tgagtacgat ggtgtggacc gtgtgatcaa tgtcaatgca        60

aagaactaca agaatgtgtt caagaagtat gaggtgctgg cactcctcta ccatgaaccc       120

cccgaggatg acaaggcctc acaaagacaa tttgagatgg aggagctgat cctggagtta 180

gcagcccaag tcctagaaga caagggtgtt ggcttcgggc tggtagactc tgagaaggat 240

gcagctgtgg ccaagaaact aggcctaact gaagtggaca gcatgtatgt attcaaggga 300

gatgaagtca ttgagtacga tggcgagttt tctgctgaca ccatcgtgga gtttctgctt 360

gatgtcctag aggaccctgt ggaattgatt gaaggtgaac gagagctgca ggcgtttgag 420

aatattgagg atgagatcaa actcattggc tacttcaaga gcaaagactc agagcattac 480

aaagccttcg aggatgcagc tgaggagttt catccctaca tccccttctt cgccaccttc 540

gacagcaagg tggcaaagaa gctgaccctg aagctgaatg agattgattt ctacgaggcc 600

ttcatggaag agcctgtgac catcccagac aagcccaata gcgaagagga gattgtcaac 660

ttcgtggagg agcacaggag atcaaccctg aggaaactga agccggagag tatgtatgag 720

acctgggagg atgatatgga tggaatccac attgtggcct tcgcagagga agctgatcct 780

gatggtttcg agttcttaga gactctcaag gctgtggccc aagataacac tgaaaaccca 840

gatcttagca tcatctggat tgaccctgat gacttccccc tgctggtccc atactgggag 900

aagacgtttg acatcgactt gtcagcccca caaataggag tcgtcaatgt tactgatgcg 960

gatagcgtat ggatggaaat ggacgatgag gaggacctgc cttctgctga ggagctggag 1020

gactggctgg aggatgtcct ggagggcgag atcaacacag aggacgatga cgatgatgat 1080

gatgac 1086

<210> 4
<211> 1143
<212> DNA
<213> Artificial Sequence

<220>
<223> CSQ2

<400> 4
gaagaggggc ttaatttccc cacatatgat gggaaggacc gagtggtaag tctttccgag 60

aagaacttca agcaggtttt aaagaaatat gacttgcttt gcctctacta ccatgagccg 120

gtgtcttcag ataaggtcac gccaaaacag ttccaactga aagaaatcgt gcttgagctt 180

gtggcccagg tccttgaaca taaagctata ggctttgtga tggtggatgc caagaaagaa 240

gccaagcttg ccaagaaact gggttttgat gaagaaggaa gcctgtatat tcttaagggt 300

gatcgcacaa tagagtttga tggcgagttt gcagctgatg tcttggtgga gttcctcttg 360

gatctaattg aagacccagt ggagatcatc agcagcaaac tggaagtcca agccttcgaa 420

cgcattgaag actacatcaa actcattggc tttttcaaga gtgaggactc agaatactac 480

aaggcttttg aagaagcagc tgaacacttc cagccttaca tcaaattctt tgccaccttt 540

gacaaagggg ttgcaaagaa attatctttg aagatgaatg aggttgactt ctatgagcca 600

tttatggatg agcccattgc catccccaac aaaccttaca cagaagagga gctggtggag 660

tttgtgaagg aacaccaaag acccactcta cgtcgcctgc gcccagaaga aatgtttgaa 720

acatgggaag atgatttgaa tgggatccac attgtggcct ttgcagagaa gagtgatcca 780

gatggctacg aattcctgga gatcctgaaa caggttgccc gggacaatac tgacaacccc 840

gatctgagca tcctgtggat cgacccggac gactttcctc tgctcgttgc ctactgggag 900

aagactttca agattgacct attcaggcca cagattgggg tggtgaatgt cacagatgct 960

gacagtgtct ggatggagat tccagatgat gacgatcttc caactgctga ggagctggag 1020

gactggattg aggatgtgct ttctggaaag ataaacactg aagatgatga tgaagatgat 1080

gatgatgatg ataattctga tgaagaggat aatgatgaca gtgatgacga tgatgatgaa 1140

tag 1143

<210> 5
<211> 7
<212> PRT
<213> Artificial Sequence

<220>
<223> TEV

<400> 5
Glu Asn Leu Tyr Phe Gln Gly
1               5

<210> 6
<211> 6
<212> PRT
<213> Artificial Sequence

<220>
<223> Thrombin

<400> 6
Leu Val Pro Arg Gly Ser
1               5

<210> 7
<211> 431
<212> PRT
<213> Artificial Sequence

<220>
<223> Ssp Dna

<400> 7
Gly Cys Ile Ser Gly Asp Ser Leu Ile Ser Leu Ala Ser Thr Gly Lys

1               5                   10                  15

Arg Val Ser Ile Lys Asp Leu Leu Asp Glu Lys Asp Phe Glu Ile Trp
            20                  25                  30

Ala Ile Asn Glu Gln Thr Met Lys Leu Glu Ser Ala Lys Val Ser Arg
        35                  40                  45

Val Phe Cys Thr Gly Lys Lys Leu Val Tyr Ile Leu Lys Thr Arg Leu
    50                  55                  60

Gly Arg Thr Ile Lys Ala Thr Ala Asn His Arg Phe Leu Thr Ile Asp
65                  70                  75                  80

Gly Trp Lys Arg Leu Asp Glu Leu Ser Leu Lys Glu His Ile Ala Leu
                85                  90                  95

Pro Arg Lys Leu Glu Ser Ser Ser Leu Gln Leu Met Ser Asp Glu Glu
            100                 105                 110

Leu Gly Leu Leu Gly His Leu Ile Gly Asp Gly Cys Thr Leu Pro Arg
        115                 120                 125

His Ala Ile Gln Tyr Thr Ser Asn Lys Ile Glu Leu Ala Glu Lys Val
    130                 135                 140

Val Glu Leu Ala Lys Ala Val Phe Gly Asp Gln Ile Asn Pro Arg Ile
145                 150                 155                 160

Ser Gln Glu Arg Gln Trp Tyr Gln Val Tyr Ile Pro Ala Ser Tyr Arg
                165                 170                 175

Leu Thr His Asn Lys Lys Asn Pro Ile Thr Lys Trp Leu Glu Asn Leu
            180                 185                 190

Asp Val Phe Gly Leu Arg Ser Tyr Glu Lys Phe Val Pro Asn Gln Val
        195                 200                 205

Phe Glu Gln Pro Gln Arg Ala Ile Ala Ile Phe Leu Arg His Leu Trp
    210                 215                 220

Ser Thr Asp Gly Cys Val Lys Leu Ile Val Glu Lys Ser Ser Arg Pro
225                 230                 235                 240

Val Ala Tyr Tyr Ala Thr Ser Ser Glu Lys Leu Ala Lys Asp Val Gln
                245                 250                 255

Ser Leu Leu Leu Lys Leu Gly Ile Asn Ala Arg Leu Ser Lys Ile Ser
            260                 265                 270

Gln Asn Gly Lys Gly Arg Asp Asn Tyr His Val Thr Ile Thr Gly Gln
        275                 280                 285

Ala Asp Leu Gln Ile Phe Val Asp Gln Ile Gly Ala Val Asp Lys Asp
    290                 295                 300

Lys Gln Ala Ser Val Glu Glu Ile Lys Thr His Ile Ala Gln His Gln
305                 310                 315                 320

Ala Asn Thr Asn Arg Asp Val Ile Pro Lys Gln Ile Trp Lys Thr Tyr
                325                 330                 335

Val Leu Pro Gln Ile Gln Ile Lys Gly Ile Thr Thr Arg Asp Leu Gln

340                 345                 350

Met Arg Leu Gly Asn Ala Tyr Cys Gly Thr Ala Leu Tyr Lys His Asn
        355                 360                 365

Leu Ser Arg Glu Arg Ala Ala Lys Ile Ala Thr Ile Thr Gln Ser Pro
    370                 375                 380

Glu Ile Glu Lys Leu Ser Gln Ser Asp Ile Tyr Trp Asp Ser Ile Val
385                 390                 395                 400

Ser Ile Thr Glu Thr Gly Val Glu Glu Val Phe Asp Leu Thr Val Pro
                405                 410                 415

Gly Pro His Asn Phe Val Ala Asn Asp Ile Ile Val His Asn Ser
            420                 425                 430

<210>    8
<211>    200
<212>    PRT
<213>    Artificial Sequence

<220>
<223>    GyrA

<400>    8
Tyr Cys Ile Thr Gly Asp Ala Leu Val Ala Leu Pro Glu Gly Glu Ser
1               5                   10                  15

Val Arg Ile Ala Asp Ile Val Pro Gly Ala Arg Pro Asn Ser Asp Asn
            20                  25                  30

Ala Ile Asp Leu Lys Val Leu Asp Arg His Gly Asn Pro Val Leu Ala
        35                  40                  45

Asp Arg Leu Phe His Ser Gly Glu His Pro Val Tyr Thr Val Arg Thr
    50                  55                  60

Val Glu Gly Leu Arg Val Thr Gly Thr Ala Asn His Pro Leu Leu Cys
65                  70                  75                  80

Leu Val Asp Val Ala Gly Val Pro Thr Leu Leu Trp Lys Leu Ile Asp
                85                  90                  95

Glu Ile Lys Pro Gly Asp Tyr Ala Val Ile Gln Arg Ser Ala Phe Ser
            100                 105                 110

Val Asp Cys Ala Gly Phe Ala Arg Gly Lys Pro Glu Phe Ala Pro Thr
        115                 120                 125

Thr Tyr Thr Val Gly Val Pro Gly Leu Val Arg Phe Leu Glu Ala His
    130                 135                 140

His Arg Asp Pro Asp Ala Gln Ala Ile Ala Asp Glu Leu Thr Asp Gly
145                 150                 155                 160

Arg Phe Tyr Tyr Ala Lys Val Ala Ser Val Thr Asp Ala Gly Val Gln
                165                 170                 175

Pro Val Tyr Ser Leu Arg Val Asp Thr Ala Asp His Ala Phe Ile Thr

180                 185                 190

Asn Gly Phe Val Ser His Asn Thr
        195                 200

<210>    9
<211>    91
<212>    PRT
<213>    Artificial Sequence

<220>
<223>    EBD14

<400>    9
Glu Val Pro Gln Leu Thr Asp Leu Ser Phe Val Asp Ile Thr Asp Ser
  1               5                  10                  15

Ser Ile Gly Leu Arg Trp Val Gln Cys Gln Cys Ala Gly Ile Ile Gly
             20                  25                  30

Tyr Arg Ile Thr Val Val Ala Ala Gly Glu Gly Ile Pro Ile Phe Glu
         35                  40                  45

Asp Phe Val Asp Pro Ala Cys Val Tyr Tyr Thr Val Thr Gly Leu Glu
     50                  55                  60

Pro Gly Ile Asp Tyr Asp Ile Ser Val Ile Thr Leu Ile His Arg Ile
 65                  70                  75                  80

His Pro Asn Pro Thr Thr Leu Thr Gln Gln Thr
                 85                  90

<210>    10
<211>    91
<212>    PRT
<213>    Artificial Sequence

<220>
<223>    EBD21

<400>    10
Glu Val Pro Gln Leu Thr Asp Leu Ser Phe Val Asp Met Thr Asp Ser
  1               5                  10                  15

Ser Ile Gly Leu Arg Trp Val Gln Val Met Arg Met Gly Ile Ile Gly
             20                  25                  30

Tyr Arg Ile Thr Val Val Ala Ala Gly Glu Gly Ile Pro Ile Phe Glu
         35                  40                  45

Asp Phe Val Asp His Pro Ile Arg Tyr Tyr Thr Val Thr Gly Leu Glu
     50                  55                  60

Pro Gly Ile Asp Tyr Asp Ile Ser Val Ile Thr Leu Ile Pro Trp Gln
 65                  70                  75                  80

Arg His Arg Pro Thr Thr Leu Thr Gln Gln Thr
                 85                  90

<210>    11
<211>    91
<212>    PRT
<213>    Artificial Sequence

<220>
<223>    EBD26


<400>    11
Glu Val Pro Gln Leu Thr Asp Leu Ser Phe Val Asp Ile Thr Asp Ser
1               5                   10                  15

Ser Ile Gly Leu Arg Trp Arg Met Thr Cys Leu Cys Leu Ile Ile Gly
            20                  25                  30

Tyr Arg Ile Thr Val Val Ala Ala Gly Glu Gly Ile Pro Ile Phe Glu
        35                  40                  45

Asp Phe Val Asp Asp Pro Trp Thr Tyr Tyr Thr Val Thr Gly Leu Glu
    50                  55                  60

Pro Gly Ile Asp Tyr Asp Ile Ser Val Ile Thr Leu Ile Thr Gln Lys
65                  70                  75                  80

His Val Lys Pro Thr Thr Leu Thr Gln Gln Thr
                85                  90


<210>    12
<211>    53
<212>    PRT
<213>    Artificial Sequence

<220>
<223>    EGF


<400>    12
Asn Ser Asp Ser Glu Cys Pro Leu Ser His Asp Gly Tyr Cys Leu His
1               5                   10                  15

Asp Gly Val Cys Met Tyr Ile Glu Ala Leu Asp Lys Tyr Ala Cys Asn
            20                  25                  30

Cys Val Val Gly Tyr Ile Gly Glu Arg Cys Gln Tyr Arg Asp Leu Lys
        35                  40                  45

Trp Trp Glu Leu Arg
    50


<210>    13
<211>    164
<212>    PRT
<213>    Artificial Sequence

<220>
<223>    KGF1


<400>    13

Met Cys Asn Asp Met Thr Pro Glu Gln Met Ala Thr Asn Val Asn Cys
1               5                   10                  15

Ser Ser Pro Glu Arg His Thr Arg Ser Tyr Asp Tyr Met Glu Gly Gly
            20                  25                  30

Asp Ile Arg Val Arg Arg Leu Phe Cys Arg Thr Gln Trp Tyr Leu Arg
        35                  40                  45

Ile Asp Lys Arg Gly Lys Val Lys Gly Thr Gln Glu Met Lys Asn Asn
    50                  55                  60

Tyr Asn Ile Met Glu Ile Arg Thr Val Ala Val Gly Ile Val Ala Ile
65                  70                  75                  80

Lys Gly Val Glu Ser Glu Phe Tyr Leu Ala Met Asn Lys Glu Gly Lys
                85                  90                  95

Leu Tyr Ala Lys Lys Glu Cys Asn Glu Asp Cys Asn Phe Lys Glu Leu
            100                 105                 110

Ile Leu Glu Asn His Tyr Asn Thr Tyr Ala Ser Ala Lys Trp Thr His
        115                 120                 125

Asn Gly Gly Glu Met Phe Val Ala Leu Asn Gln Lys Gly Ile Pro Val
    130                 135                 140

Arg Gly Lys Lys Thr Lys Lys Glu Gln Lys Thr Ala His Phe Leu Pro
145                 150                 155                 160

Met Ala Ile Thr

<210>   14
<211>   147
<212>   PRT
<213>   Artificial Sequence

<220>
<223>   VEGF

<400>   14
Met Asn Phe Leu Leu Ser Trp Val His Trp Ser Leu Ala Leu Leu Leu
1               5                   10                  15

Tyr Leu His His Ala Lys Trp Ser Gln Ala Ala Pro Met Ala Glu Gly
            20                  25                  30

Gly Gly Gln Asn His His Glu Val Val Lys Phe Met Asp Val Tyr Gln
        35                  40                  45

Arg Ser Tyr Cys His Pro Ile Glu Thr Leu Val Asp Ile Phe Gln Glu
    50                  55                  60

Tyr Pro Asp Glu Ile Glu Tyr Ile Phe Lys Pro Ser Cys Val Pro Leu
65                  70                  75                  80

Met Arg Cys Gly Gly Cys Cys Asn Asp Glu Gly Leu Glu Cys Val Pro
                85                  90                  95

Thr Glu Glu Ser Asn Ile Thr Met Gln Ile Met Arg Ile Lys Pro His

100                 105                 110

Gln Gly Gln His Ile Gly Glu Met Ser Phe Leu Gln His Asn Lys Cys
        115                 120                 125

Glu Cys Arg Pro Lys Lys Asp Arg Ala Arg Gln Glu Lys Cys Asp Lys
    130                 135                 140

Pro Arg Arg
145

<210>    15
<211>    146
<212>    PRT
<213>    Artificial Sequence

<220>
<223>    FGF2

<400>    15
Pro Ala Leu Pro Glu Asp Gly Gly Ser Gly Ala Phe Pro Pro Gly His
1               5                   10                  15

Phe Lys Asp Pro Lys Arg Leu Tyr Cys Lys Asn Gly Gly Phe Phe Leu
            20                  25                  30

Arg Ile His Pro Asp Gly Arg Val Asp Gly Val Arg Glu Lys Ser Asp
        35                  40                  45

Pro His Ile Lys Leu Gln Leu Gln Ala Glu Glu Arg Gly Val Val Ser
    50                  55                  60

Ile Lys Gly Val Cys Ala Asn Arg Tyr Leu Ala Met Lys Glu Asp Gly
65                  70                  75                  80

Arg Leu Leu Ala Ser Lys Cys Val Thr Asp Glu Cys Phe Phe Phe Glu
                85                  90                  95

Arg Leu Glu Ser Asn Asn Tyr Asn Thr Tyr Arg Ser Arg Lys Tyr Thr
            100                 105                 110

Ser Trp Tyr Val Ala Leu Lys Arg Thr Gly Gln Tyr Lys Leu Gly Ser
        115                 120                 125

Lys Thr Gly Pro Gly Gln Lys Ala Ile Leu Phe Leu Pro Met Ser Ala
    130                 135                 140

Lys Ser
145

<210>    16
<211>    115
<212>    PRT
<213>    Artificial Sequence

<220>
<223>    BMP2

<400>    16

Met Gln Ala Lys His Lys Gln Arg Lys Arg Leu Lys Ser Ser Cys Lys
1               5                   10                  15

Arg His Pro Leu Tyr Val Asp Phe Ser Asp Val Gly Trp Asn Asp Trp
            20                  25                  30

Ile Val Ala Pro Pro Gly Tyr His Ala Phe Tyr Cys His Gly Glu Cys
        35                  40                  45

Pro Phe Pro Leu Ala Asp His Leu Asn Ser Thr Asn His Ala Ile Val
    50                  55                  60

Gln Thr Leu Val Asn Ser Val Asn Ser Lys Ile Pro Lys Ala Cys Cys
65                  70                  75                  80

Val Pro Thr Glu Leu Ser Ala Ile Ser Met Leu Tyr Leu Asp Glu Asn
                85                  90                  95

Glu Lys Val Val Leu Lys Asn Tyr Gln Asp Met Val Val Glu Gly Cys
            100                 105                 110

Gly Cys Arg
        115


<210>   17
<211>   113
<212>   PRT
<213>   Artificial Sequence

<220>
<223>   TGF


<400>   17
Ala Leu Asp Thr Asn Tyr Cys Phe Ser Ser Thr Glu Lys Asn Cys Cys
1               5                   10                  15

Val Arg Gln Leu Tyr Ile Asp Phe Arg Lys Asp Leu Gly Trp Lys Trp
            20                  25                  30

Ile His Glu Pro Lys Gly Tyr His Ala Asn Phe Cys Leu Gly Pro Cys
        35                  40                  45

Pro Tyr Ile Trp Ser Leu Asp Thr Gln Tyr Ser Lys Val Leu Ala Leu
    50                  55                  60

Tyr Asn Gln His Asn Pro Gly Ala Ser Ala Ala Pro Cys Cys Val Pro
65                  70                  75                  80

Gln Ala Leu Glu Pro Leu Pro Ile Val Tyr Tyr Val Gly Arg Lys Pro
                85                  90                  95

Lys Val Glu Gln Leu Ser Asn Met Ile Val Arg Ser Cys Lys Cys Ser
            100                 105                 110

Arg


<210>   18
<211>   323
<212>   PRT

<213>    Artificial Sequence

<220>
<223>    HRP

<400>    18
Gln Leu Thr Pro Thr Phe Tyr Asp Asn Ser Cys Pro Asn Val Ser Asn
1               5                   10                  15

Ile Val Arg Asp Thr Ile Val Asn Glu Leu Arg Ser Asp Pro Arg Ile
            20                  25                  30

Ala Ala Ser Ile Leu Arg Leu His Phe His Asp Cys Phe Val Asn Gly
        35                  40                  45

Cys Asp Ala Ser Ile Leu Leu Asp Asn Thr Thr Ser Phe Arg Thr Glu
    50                  55                  60

Lys Asp Ala Phe Gly Asn Ala Asn Ser Ala Arg Gly Phe Pro Val Ile
65                  70                  75                  80

Asp Arg Met Lys Ala Ala Val Glu Ser Ala Cys Pro Arg Thr Val Ser
                85                  90                  95

Cys Ala Asp Leu Leu Thr Ile Ala Ala Gln Gln Ser Val Thr Leu Ala
            100                 105                 110

Gly Gly Pro Ser Trp Arg Val Pro Leu Gly Arg Arg Asp Ser Leu Gln
        115                 120                 125

Ala Phe Leu Asp Leu Ala Asn Ala Asn Leu Pro Ala Pro Phe Phe Thr
    130                 135                 140

Leu Pro Gln Leu Lys Asp Ser Phe Arg Asn Val Gly Leu Asn Arg Ser
145                 150                 155                 160

Ser Asp Leu Val Ala Leu Ser Gly Gly His Thr Phe Gly Lys Asn Gln
                165                 170                 175

Cys Arg Phe Ile Met Asp Arg Leu Tyr Asn Phe Ser Asn Thr Gly Leu
            180                 185                 190

Pro Asp Pro Thr Leu Asn Thr Thr Tyr Leu Gln Thr Leu Arg Gly Leu
        195                 200                 205

Cys Pro Leu Asn Gly Asn Leu Ser Ala Leu Val Asp Phe Asp Leu Arg
    210                 215                 220

Thr Pro Thr Ile Phe Asp Asn Lys Tyr Tyr Val Asn Leu Glu Glu Gln
225                 230                 235                 240

Lys Gly Leu Ile Gln Ser Asp Gln Glu Leu Phe Ser Ser Pro Asn Ala
                245                 250                 255

Thr Asp Thr Ile Pro Leu Val Arg Ser Phe Ala Asn Ser Thr Gln Thr
            260                 265                 270

Phe Phe Asn Ala Phe Val Glu Ala Met Asp Arg Met Gly Asn Ile Thr
        275                 280                 285

Pro Leu Thr Gly Thr Gln Gly Gln Ile Arg Leu Asn Cys Arg Val Val
    290                 295                 300

Asn Ser Asn Ser Leu Leu His Asp Met Val Glu Val Val Asp Phe Val
305                 310                 315                 320

Ser Ser Met

<210>    19
<211>    32
<212>    PRT
<213>    Artificial Sequence

<220>
<223>    GLP1

<400>    19
Ala Ala His Gly Glu Gly Thr Phe Thr Ser Asp Val Ser Ser Tyr Leu
  1               5                  10                  15

Glu Glu Gln Ala Ala Lys Glu Phe Ile Ala Trp Leu Val Lys Gly Ala
            20                  25                  30

<210>    20
<211>    30
<212>    DNA
<213>    Artificial Sequence

<220>
<223>    PRIMER

<400>    20
aatggatccg aggtgcccca actcactgac                                    30

<210>    21
<211>    36
<212>    DNA
<213>    Artificial Sequence

<220>
<223>    PRIMER

<400>    21
attctcgagt tacgtttgtt gtgtcagtgt agtagg                             36

<210>    22
<211>    30
<212>    DNA
<213>    Artificial Sequence

<220>
<223>    PRIMER

<400>    22

aatggatcca actctgatag cgaatgcccg 30

<210> 23
<211> 30
<212> DNA
<213> Artificial Sequence

<220>
<223> PRIMER

<400> 23
attctcgagt taacgcagtt cccaccattt 30

<210> 24
<211> 21
<212> DNA
<213> Artificial Sequence

<220>
<223> PRIMER

<400> 24
cagcaagaca gcgatggatc c 21

<210> 25
<211> 21
<212> DNA
<213> Artificial Sequence

<220>
<223> PRIMER

<400> 25
cgacttacag gtgatctcga g 21

## Description

### Technical Field

The present disclosure relates to a method for expression, water solubilization, and purification of proteins via a CSQ-tag (calsequestrin-tag) and provides a fusion protein comprising a CSQ-tag and a protein of interest, a nucleic acid including a nucleotide sequence coding for the fusion protein, an expression vector carrying the nucleic acid, a cell transformed with the expression vector, and a method for expressing, water-solubilizing, and purifying the protein of interest by using the CSQ tag. Designed to improve the expression and water solubilization of proteins widely used in pharmaceuticals and cosmetics through a CSQ-tag and to easily segregate the proteins with the aid of calcium, the present disclosure is expected to decrease production costs of protein substances and protein pharmaceuticals.

### Background Art

With the technical advance of the genetic engineering and biology science, a great number of trials have recently been made to produce or obtain specific proteins on a mass scale and to apply the same to various industries and disease therapies. In this context, focus has been paid to the development of protein recombination, mass production, and purification technologies so as to acquire proteins of interest. For the most part, proteins of interest can be obtained through expression by culturing cells transformed with vectors which carry genes coding for the proteins of interest. In many cases, proteins can be expressed in eukaryotes, prokaryotes, etc. For some special cases, the expression may be achieved in transgenic plants or animals. By way of example, a trial may be made to express a protein of interest in a transgenic mammalian animal and to produce the same through the milk secreted therefrom. In this regard, the protein of interest can be separated and purified from the cell culture or milk.

When expression is carried out in plants, animals, or microorganisms which lack an additional strategy for obtaining a protein of interest through secretion, a process of extracting the protein from the storage organ or the inside of cells is required. However, the work of obtaining a protein of interest from transformed cells is not easy. Hence, a protein of interest is not produced in a native form, but now frequently in a recombinant form including a tag which is intended to facilitate protein purification. The use of tags in protein purification is a technique which is very highly efficient among various protein purification techniques. The tags available for the purpose are divided into peptide tags and protein tags. Peptide tags are composed of short amino acid sequences. Representative of the peptide tags is a His-tag (histidine-tag), especially, a hexahistidine tag (His6-tag). Since histidine peptides have specific chemical affinity for nickel, fusion proteins including corresponding tags can be purified at high purity by a nickel-containing column. A protein tag, which is designed to utilize a protein domain binding to a specific ingredient, includes the corresponding protein domain therein. A GST-tag (glutathione S-transferase-tag) is representative of protein tags. A GST tag can be purified at high purity by a column employing the GST substrate glutathione as a fixing mediator.

Leading to the present disclosure, intensive and thorough research, conducted by the present inventors, into the development of a novel tag facilitating the acquirement of a protein of interest, resulted in the finding that when fused with a CSQ (calsequestrin) tag, a protein of interest can be easily expressed, water-solubilized, and purified.

### [Related Art Document]

Korean Patent Number 10-2014-0026781 A

### Disclosure of Invention

### Technical Problem

An aspect of the present disclosure provides a fusion protein including a CSQ-tag and a protein of interest.

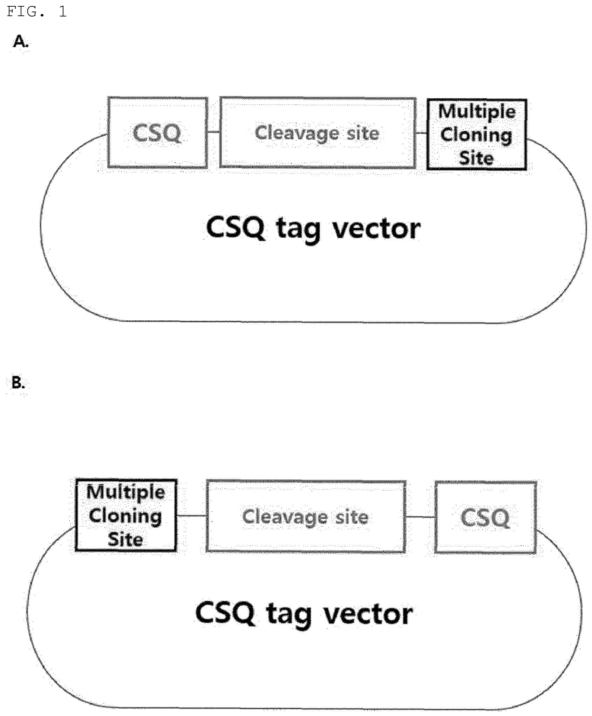

Another aspect of the present disclosure provides a nucleic acid including a nucleotide sequence coding for the fusion protein, and an expression vector carrying the nucleic acid. A schematic view of a recombinant expression vector according to the present disclosure is given in FIG. 1.

A further aspect of the present disclosure provides a cell transformed with the expression vector.

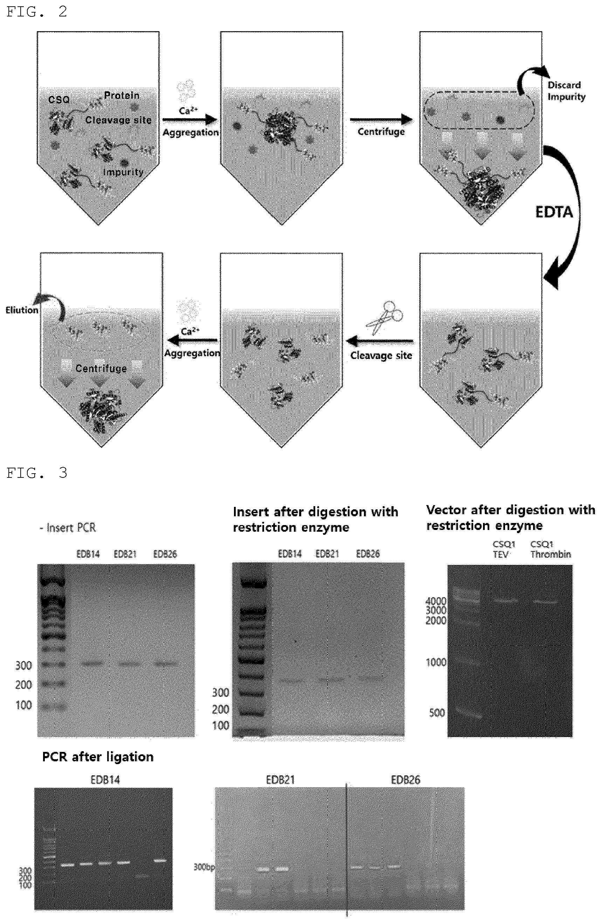

A still further aspect of the present disclosure provides a method for expressing and purifying a protein of interest, using a CSQ tag. The method for expressing and purifying a protein of interest according to the present disclosure is exemplified as seen in FIG. 2.

### Solution to Problem

The present disclosure pertains to a fusion protein including a CSQ tag and a protein of interest, wherein the CSQ tag acts to improve the expression and water solubility of the protein of interest.

The CSQ tag may be coded for by an amino acid sequence of SEQ ID NO: 1 or 2.

The CSQ tag may be encoded by a nucleotide sequence of SEQ ID NO: 4 or 4.

The CSQ tag and the protein of interest may be fused to each other via a hydrolase-cleavable peptide composed of an amino acid sequence selected from the group consisting of SEQ ID NOS: 5 to 8.

The protein of interest is selected from the group consisting of a polymer protein, a glycoprotein, a cytokine, a growth factor, a blood factor, a vaccine, a hormone, an enzyme, and an antibody. For example, the protein of interest may be selected from the group consisting of interleukin-2, blood clotting factor VII, blood clotting factor VIII, blood clotting factor IX, an immunoglobulin, horseradish peroxidase (HRP), a cytokine, α-interferon, β-interferon, γ-interferon, colony stimulating factor (GM-CSF), human fibronectin extra domain B (EBD), bone morphogenetic protein (BMP), fibroblast growth factor (FGF), vascular endothelial growth factor (VEGF), nerve growth factor (NGF), epidermal growth factor (EGF), insulin-like growth factor (IGF), trans-forming growth factor-α and -β (TGF-α and -β), brain-derived neurotrophic factor (BDNF), platelet-derived growth factor (PDGF), placental growth factor (PlGF), hepatocyte growth factor (HGF), fibroblast growth factor 1 and 2 (FGF-1 and -2), keratinocyte growth factor (KGF), glucagon-like peptide-1 (GLP-1), exendin, somatostatin, LHRH (luteinizing hormone-releasing hormone), adrenocorticotropic hormone, growth hormone-releasing hormone, oxytocin, thymosin alpha-1, corticotropin-releasing factor, calcitonin, bivalirudin, vasopressin, phospholipase-activating protein (PLAP), insulin, tumor necrosis factor (TNF), follicle-stimulating hormone, thyroid-stimulating hormone, antidiuretic hormone, pigmentary hormone, parathyroid hormone, luteinizing hormone, calcitonin gene-related peptide (CGRP), enkephalin, somatomedin, erythropoietin, hypothalamic releasing factor, prolactin, chorionic gonadotropin, tissue plasminogen activator, growth hormone releasing peptide (GHPR), thymic humoral factor (THF), asparaginase, arginase, arginine deiminase, adenosine deaminase, peroxidase dismutase, endotoxinase, catalase, chymotrypsin, lipase, uricase, adenosine diphosphatase, tyrosinase, bilirubin oxidase, glucose oxidase, glucosidase, galactosidase, glucocerebrosidase, and glucuronidase.

The protein of interest may be coded for by an amino acid sequence selected from the group consisting of SEQ ID NOS: 9 to 19.

Also, the present disclosure pertains to a nucleic acid including a nucleotide sequence encoding the fusion protein.

Also, the present disclosure pertains to an expression vector carrying the nucleic acid.

Also, the present disclosure pertains to a cell transformed with the expression vector.

The cell may be Escherichia coli, Bacillus subtilis, Bacillus thuringiensis, Salmonella typhimurium, Serratia marcescens, Pseudomonas spp. yeast, insect cells, CHO (Chinese hamster ovary) cells, W138, BHK, COS-7, 293, HepG2, 3T3, RIN, MDCK cells, or plant cells.

The present disclosure pertains to a method for expressing and purifying a protein of interest by using a CSQ tag, the method comprising the steps of constructing an expressing vector carrying a nucleic acid coding for the fusion protein;
B) transducing the expression vector into a host cell to obtain a transformant;
C) expressing the fusion protein including the CSQ tag and the protein of interest in the transformant;
D) precipitating the fusion protein including the CSQ tag and the protein of interest from the transformant with the aid of calcium; and
E) separating the protein of interest from the fusion protein with a hydrolase.

### Advantageous Effects of Invention

According to the method for expressing, water-solubilizing, and purifying a protein of interest by using CSQ tag of the present disclosure, a protein that is unlikely to express in an aqueous state or is likely to aggregate in an aqueous condition can be expressed in an aqueous state due to the high expression and water solubility of CSQ and can be separated and purified in a column-less manner by precipitation with calcium. Thus, the present disclosure is expected to lower production costs of protein substances and protein pharmaceuticals.

### Brief Description of Drawings

FIG. 1 shows schematic diagrams of recombinant expression vectors according to the present disclosure.
FIG. 2 is a schematic view illustrating an expression and purification method for a protein of interest according to the present disclosure.
FIG. 3 shows data of cloning a gene coding for the protein of interest EDB into CSQ1TEV or CSQ1Thrombin vector according to Example 1.

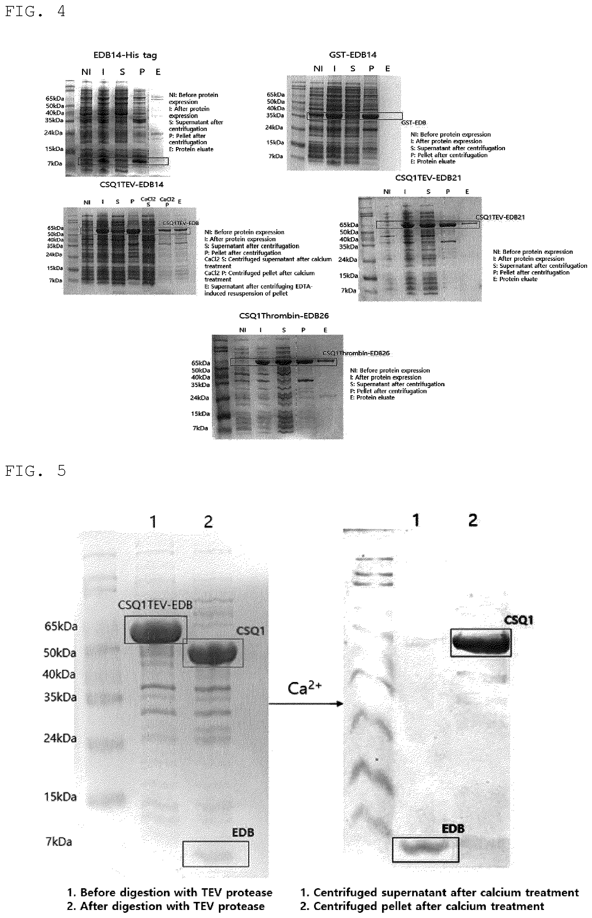

FIG. 4 shows purification results of CSQ1TEV-EDB and CSQ1Thrombin-EDB proteins according to Example 1 in comparison with purification of EDB protein as fusions with the conventional commercially available tags His tag and GST. Although EDB14 protein can be expressed using His-tag or GST-tag, the protein of interest is not detected in a supernatant, but aggregates into an insoluble pellet. However, when expressed by using CSQ-tag, EDB14, 21, and 26 proteins, although aggregating in part into pellets, exist as soluble forms at a rate of about 30-50% in the supernatant (S). Thus, the data imply that it is possible to elute a protein of interest.
FIG. 5 shows results of digesting CSQ1TEV-EDB protein with TEV protease according to Example 1. After CSQ-tag and EDB protein were separated from each other by TEV protease, the application of calcium precipitates the separated CSQ-tag so that the protein of interest EDB can be easily purified.
FIG. 6 shows data of cloning a gene coding for the protein of interest EGF into CSQ1TEV or CSQ1Thrombin vector according to Example 2.
FIG. 7 shows data of purifying CSQ1Thrombin-EGF and CSQ1TEV-EGF proteins according to Example 2. EGF expressed in bacterial cells is known to exist in the form of pellets at a rate of 100%. However, the use of CSQ-tag is shown to make the protein soluble at a rate of 30-40%.
FIG. 8 shows data of digesting CSQ1Thrombin-EGF protein with thrombin protease according to Example 2.
FIG. 9 shows data of purifying CSQ1TEV-KGF1 protein according to Example 3.
FIG. 10 shows data of digesting CSQ1TEV-KGF1 with TEV protease according to Example 3.
FIG. 11 shows data of purifying CSQ1TEV-VEGF protein according to Example 3.
FIG. 12 shows data of digesting CSQ1TEV-VEGF protein with TEV protease according to Example 3.
FIG. 13 shows data of purifying CSQ1TEV-FGF2 protein according to Example 3.
FIG. 14 shows data of digesting CSQ1TEV-FGF2 protein with TEV protease according to Example 3.
FIG. 15 shows data of cloning genes coding for the proteins of interest BMP2 and TGFβ into TEVCSQ1 or ThrombinCSQ1 vector according to Example 4.
FIG. 16 shows data of purifying BMP2-TEVCSQ1 and BMP2-ThrombinCSQ1 proteins according to Example 4.
FIG. 17 shows data of digesting BMP2-TEVCSQ1 or BMP2-ThrombinCSQ1 protein with TEV protease or Thrombin protease according to Example 4.
FIG. 18 shows data of purifying TGFβ-TEVCSQ1 and TGFβ-ThrombinCSQ1 proteins according to Example 4.
FIG. 19 shows data of digesting TGFβ-TEVCSQ1 or TGFβ-ThrombinCSQ1 protein with TEV protease or Thrombin protease according to Example 4.
FIG. 20 shows data of purifying HRP-TEVCSQ1 protein according to Example 4.
FIG. 21 shows data of digesting HRP-TEVCSQ1 protein with TEV protease according to Example 4.
FIG. 22 shows data of purifying GLP1-TEVCSQ1 protein according to Example 4.
FIG. 23 shows data of digesting GLP1-TEVCSQ1 protein with TEV protease according to Example 4.
FIG. 24 shows data of cloning genes coding for the proteins of interest BMP2 and KGF1 into CSQ2TEV or CSQ2Thrombin vector according to Example 5.
FIG. 25 shows data of purifying CSQ2TEV-BMP2 and CSQ2Thrombin-BMP2 proteins according to Example 5.
FIG. 26 shows data of digesting CSQ2TEV-BMP2 or CSQ2Thrombin-BMP2 protein with TEV protease or Thrombin protease according to Example 5.
FIG. 27 shows data of purifying CSQ2TEV-KGF1 and CSQ2Thrombin-KGF1 proteins according to Example 5.
FIG. 28 shows data of digesting CSQ2TEV-KGF1 or CSQ2Thrombin-KGF1 protein with TEV protease or Thrombin protease according to Example 5.
FIG. 29 shows data of purifying HRP-TEVCSQ2 and HRP-ThrombinCSQ2 proteins according to Example 6.
FIG. 30 shows data of digesting HRP-TEVCSQ2 protein with TEV protease according to Example 6.
FIG. 31 shows data of purifying GLP1-TEVCSQ2 protein according to Example 6.
FIG. 32 shows data of digesting GLP1-TEVCSQ2 protein with TEV protease according to Example 6.
FIG. 33 shows data of purifying CSQ1-Ssp Dna-EGF protein according to Example 7.
FIG. 34 shows data of cleaving CSQ1-Ssp Dna-EGF protein with dithiothreitol (DTT) according to Example 7.
FIG. 35 shows data of purifying CSQ2-Ssp Dna-KGF1 protein according to Example 7.
FIG. 36 shows data of cleaving CSQ2-Ssp Dna-KGF1 protein with dithiothreitol (DTT) according to Example 7.
FIG. 37 shows data of purifying EGF-GyrA-CSQ1 protein according to Example 8.
FIG. 38 shows data of cleaving EGF-GyrA-CSQ1 protein with 20mM Na-HEPES (pH 6.5) buffer according to Example 8.
FIG. 39 shows data of purifying BMP2-GyrA-CSQ2 protein according to Example 8.

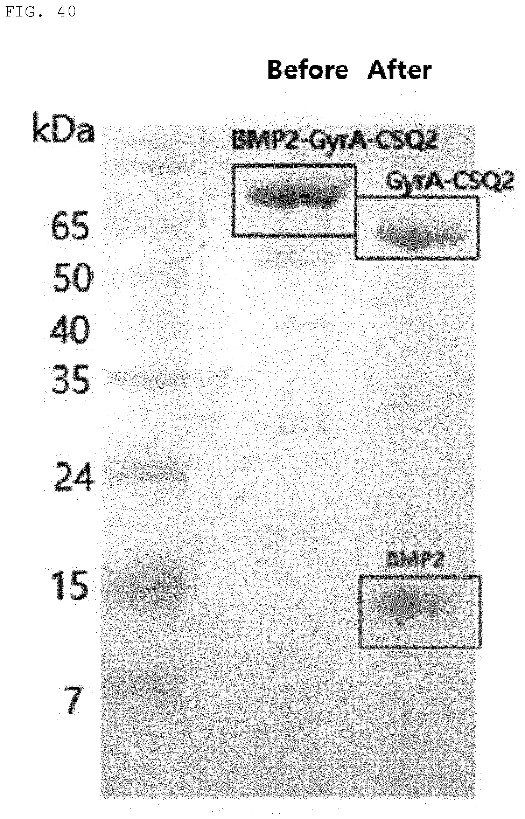

FIG. 40 shows data of cleavage with 20mM Na-HEPES (pH 6.5) buffer according to Example 8.

### Best Mode for Carrying out the Invention

The term "calsequestrin" or "CSQ", as used herein, refers to a calcium-binding protein that acts as a calcium buffer within the sarcoplasmic reticulum and helps hold calcium in the cisterna of the sarcoplasmic reticulum by binding calcium after a muscle contraction, even though the contraction of calcium in the sarcoplasmic reticulum is much higher than in the cytosol. Each molecule of calsequestrin can bind many calcium ions (e.g., one molecule of calsequestrin has 40-50 calcium-binding sites) and as such, exhibits a very high capability of storing calcium. Calsequestrin exists as monomers at a low concentration of calcium and forms a polymer and increases in size as calcium concentration is increased.

As used herein, the term "protein of interest" refers to a protein that is required to be obtained at high purity or in a large amount according to a specific purpose and is intended to encompass any native protein, variant protein, or novel recombinant protein, with not limitations thereto. A protein of interest may be a protein that is required at high purity or in a large amount for industrial, medical, or scientific reason, or other reasons and particularly, may be a recombinant protein for medical or academic studies and more particularly, may be selected from the group consisting of a polymer protein, a glycoprotein, a cytokine, a growth factor, a blood factor, a vaccine, a hormone, an enzyme, and an antibody. The protein of interest may be far more particularly an entirety or portion of a light or a heavy chain of an antibody and most particularly a light-chain variable region (VL) or a heavy-chain variable region (VH) of an antibody.

As used herein, the term "hydrolase-cleavable peptide" refers to a peptide that can be cleaved by a hydrolase. The hydrolase-cleavable peptide may be located between a CSQ tag and a protein of interest and is cleaved by a hydrolase to separate the CSQ tag and the protein of interest.

As used herein, the term "vector" is an expression vector that allows the expression of a protein of interest in a suitable host cell and refers to a nucleic acid construct including essential regulatory elements operably linked to express a nucleic acid insert. So long as it is usually used, any plasmid may be used as the vector. Examples of the plasmid include bacterial plasmids, phages, yeast plasmids, plant cell viruses, mammalian cell viruses, and other vectors. The vector may be constructed by manipulating vectors usually used in the art, such as plasmids (e.g., pSC101, pGV1106, pACYC177, ColE1, pKT230, pME290, pBR322, pUC8/9, pUC6, pBD9, pHC79, pIJ61, pLAFR1, pHV14, pGEX series, pET series, and pUC19), phages (e.g., λgt4λB, λ-Charon, λΔz1, M13, etc.), or viruses (e.g., CMV, SV40, etc.).

As used herein, the term "transformation" means introduction of DNA into a host cell so that the DNA is replicable either as an extra-chromosomal element or by chromosomal integration and refers to artificial genetic alteration by introducing a foreign DNA into a host cell. Examples of the transformation available in the present disclosure include, but are not limited to, CaCl2 precipitation, a Hanahan method, which employs DMSO (dimethyl sulfoxide) as a reducing material in combination with CaCl2 precipitation so as to improve the efficiency, electroporation, calcium phosphate precipitation, protoplast fusion, vortexing in the presence of silicon carbide fibers, agrobacterium-mediated transformation, PEG-mediated transformation, dextran sulfate-, lipofectamine-, and desiccation/inhibition-mediated transformation.

As used herein, the term "host cell" refers to a cell that allows a guest, such as a different microbe or gene to be introduced thereinto and supplies nutrients to the guest. In this context, the host cell means a cell transformed with a vector wherein the vector exhibits various genetic or molecular influences. So long as it allows the vector of the present disclosure to be cloned and expressed stably and continuously therein, any host cells known in the art may be employed in the present disclosure. Examples of available prokaryotic host cells include *E. coli Rosetta, E. coli JM109, E. coli BL21, E. coli RR1, E. coli LE392, E. coli B, E. coli X 1776, E. coli W3110,* Bacillus spp. such as Bacillus subtilis and Bacillus thuringiensis, and gut bacteria such as Salmonella typhimurium, Serratia marcescens, and various Pseudomonas spp. As eukaryotic host cells into which the vector of the present disclosure is transformed, yeast (*Saccharomyce cerevisiae*)*,* insect cells, human cells (e.g., CHO (Chinese hamster ovary) cells, W138, BHK, COS-7, 293, HepG2, 3T3, RIN, and MDCK cells), and plant cells may be used.

According to a first embodiment, the present disclosure provides a fusion protein comprising a CSQ tag and a protein of interest.

In the fusion protein according to the present disclosure, the CSQ tag includes CSQ1 or CSQ2.

In the fusion protein according to the present disclosure, the CSQ1 and the CSQ2 may be coded for by amino acid sequences of SEQ ID NOS: 1 and 2, respectively. The amino acid sequence of SEQ ID NO: 1 may be encoded by the nucleotide sequence of SEQ ID NO: 3 and the amino acid sequence of SEQ ID NO: 2 may be encoded by the nucleotide sequence of SEQ ID NO: 4.

In the fusion protein according to the present disclosure, the CSQ tag and the protein of interest may be fused to each other via a hydrolase-cleavable peptide composed of an amino acid sequence selected from the group consisting of SEQ ID NOS: 5 to 8.

In the fusion protein according to the present disclosure, the protein of interest is selected from the group consisting of a polymer protein, a glycoprotein, a cytokine, a growth factor, a blood factor, a vaccine, a hormone, an enzyme, and an antibody. For example, the protein of interest may be selected from the group consisting of interleukin-2, blood clotting factor VII, blood clotting factor VIII, blood clotting factor IX, an immunoglobulin, horseradish peroxidase (HRP), a cytokine, α-interferon, β-interferon, γ-interferon, colony stimulating factor (GM-CSF), human fibronectin extra domain B (EBD), bone morphogenetic protein (BMP), fibroblast growth factor (FGF), vascular endothelial growth factor (VEGF), nerve growth factor (NGF), epidermal growth factor (EGF), insulin-like growth factor (IGF), trans-forming growth factor-α and -β (TGF-α and -β), brain-derived neurotrophic factor (BDNF), platelet-derived growth factor (PDGF), placental growth factor (PlGF), hepatocyte growth factor (HGF), fibroblast growth factor 1 and 2 (FGF-1 and -2), keratinocyte growth factor (KGF), glucagon-like peptide-1 (GLP-1), exendin, somatostatin, LHRH (luteinizing hormone-releasing hormone), adrenocorticotropic hormone, growth hormone-releasing hormone, oxytocin, thymosin alpha-1, corticotropin-releasing factor, calcitonin, bivalirudin, vasopressin, phospholipase-activating protein (PLAP), insulin, tumor necrosis factor (TNF), follicle-stimulating hormone, thyroid-stimulating hormone, antidiuretic hormone, pigmentary hormone, parathyroid hormone, luteinizing hormone, calcitonin gene-related peptide (CGRP), enkephalin, somatomedin, erythropoietin, hypothalamic releasing factor, prolactin, chorionic gonadotropin, tissue plasminogen activator, growth hormone releasing peptide (GHPR), thymic humoral factor (THF), asparaginase, arginase, arginine deiminase, adenosine deaminase, peroxidase dismutase, endotoxinase, catalase, chymotrypsin, lipase, uricase, adenosine diphosphatase, tyrosinase, bilirubin oxidase, glucose oxidase, glucosidase, galactosidase, glucocerebrosidase, and glucuronidase.

In the fusion protein according to the present disclosure, the protein of interest may be coded for by an amino acid sequence selected from the group consisting of SEQ ID NOS: 9 to 19.

According to a second embodiment, the present disclosure provides a nucleic acid coding for a fusion protein including a CSQ tag and a protein of interest, and an expression vector carrying the nucleic acid.

In the nucleic acid or expression vector according to the present disclosure, the CSQ tag may be coded for by an amino acid sequence of SEQ ID NO: 1 or 2. The amino acid sequence of SEQ ID NO: 1 may be encoded by the nucleotide sequence of SEQ ID NO: 3 and the amino acid sequence of SEQ ID NO: 2 may be encoded by the nucleotide sequence of SEQ ID NO: 4.

In the nucleic acid or expression vector according to the present disclosure, the CSQ tag and the protein of interest may be fused to each other via a hydrolase-cleavable peptide composed of an amino acid sequence selected from the group consisting of SEQ ID NOS: 5 to 8.

In the nucleic acid or expression vector according to the present disclosure, the protein of interest is selected from the group consisting of a polymer protein, a glycoprotein, a cytokine, a growth factor, a blood factor, a vaccine, a hormone, an enzyme, and an antibody. For example, the protein of interest may be selected from the group consisting of interleukin-2, blood clotting factor VII, blood clotting factor VIII, blood clotting factor IX, an immunoglobulin, horseradish peroxidase (HRP), a cytokine, α-interferon, β-interferon, γ-interferon, colony stimulating factor (GM-CSF), human fibronectin extra domain B (EBD), bone morphogenetic protein (BMP), fibroblast growth factor (FGF), vascular endothelial growth factor (VEGF), nerve growth factor (NGF), epidermal growth factor (EGF), insulin-like growth factor (IGF), trans-forming growth factor-α and -β (TGF-α and -β), brain-derived neurotrophic factor (BDNF), platelet-derived growth factor (PDGF), placental growth factor (PlGF), hepatocyte growth factor (HGF), fibroblast growth factor 1 and 2 (FGF-1 and -2), keratinocyte growth factor (KGF), glucagon-like peptide-1 (GLP-1), exendin, somatostatin, LHRH (luteinizing hormone-releasing hormone), adrenocorticotropic hormone, growth hormone-releasing hormone, oxytocin, thymosin alpha-1, corticotropin-releasing factor, calcitonin, bivalirudin, vasopressin, phospholipase-activating protein (PLAP), insulin, tumor necrosis factor (TNF), follicle-stimulating hormone, thyroid-stimulating hormone, antidiuretic hormone, pigmentary hormone, parathyroid hormone, luteinizing hormone, calcitonin gene-related peptide (CGRP), enkephalin, somatomedin, erythropoietin, hypothalamic releasing factor, prolactin, chorionic gonadotropin, tissue plasminogen activator, growth hormone releasing peptide (GHPR), thymic humoral factor (THF), asparaginase, arginase, arginine deiminase, adenosine deaminase, peroxidase dismutase, endotoxinase, catalase, chymotrypsin, lipase, uricase, adenosine diphosphatase, tyrosinase, bilirubin oxidase, glucose oxidase, glucosidase, galactosidase, glucocerebrosidase, and glucuronidase.

In the nucleic acid or expression vector according to the present disclosure, the protein of interest may be coded for by an amino acid sequence selected from the group consisting of SEQ ID NOS: 9 to 19.

According to a third embodiment, the present disclosure provides a cell transformed with an expression vector carrying a nucleic acid coding for a fusion protein including a CSQ tag and a protein of interest.

In the transformed cell according to the present disclosure, the CSQ tag may be coded for by an amino acid sequence of SEQ ID NO: 1 or 2. The amino acid sequence of SEQ ID NO: 1 may be encoded by the nucleotide sequence of SEQ ID NO: 3 and the amino acid sequence of SEQ ID NO: 2 may be encoded by the nucleotide sequence of SEQ ID NO: 4.

In the transformed cell according to the present disclosure, the CSQ tag and the protein of interest may be fused to each other via a hydrolase-cleavable peptide composed of an amino acid sequence selected from the group consisting of SEQ ID NOS: 5 to 8.

In the transformed cell according to the present disclosure, the protein of interest is selected from the group consisting of a polymer protein, a glycoprotein, a cytokine, a growth factor, a blood factor, a vaccine, a hormone, an enzyme, and an antibody. For example, the protein of interest may be selected from the group consisting of interleukin-2, blood clotting factor VII, blood clotting factor VIII, blood clotting factor IX, an immunoglobulin, horseradish peroxidase (HRP), a cytokine, α-interferon, β-interferon, γ-interferon, colony stimulating factor (GM-CSF), human fibronectin extra domain B (EBD), bone morphogenetic protein (BMP), fibroblast growth factor (FGF), vascular endothelial growth factor (VEGF), nerve growth factor (NGF), epidermal growth factor (EGF), insulin-like growth factor (IGF), trans-forming growth factor-α and -β (TGF-α and -β), brain-derived neurotrophic factor (BDNF), platelet-derived growth factor (PDGF), placental growth factor (PlGF), hepatocyte growth factor (HGF), fibroblast growth factor 1 and 2 (FGF-1 and -2), keratinocyte growth factor (KGF), glucagon-like peptide-1 (GLP-1), exendin, somatostatin, LHRH (luteinizing hormone-releasing hormone), adrenocorticotropic hormone, growth hormone-releasing hormone, oxytocin, thymosin alpha-1, corticotropin-releasing factor, calcitonin, bivalirudin, vasopressin, phospholipase-activating protein (PLAP), insulin, tumor necrosis factor (TNF), follicle-stimulating hormone, thyroid-stimulating hormone, antidiuretic hormone, pigmentary hormone, parathyroid hormone, luteinizing hormone, calcitonin gene-related peptide (CGRP), enkephalin, somatomedin, erythropoietin, hypothalamic releasing factor, prolactin, chorionic gonadotropin, tissue plasminogen activator, growth hormone releasing peptide (GHPR), thymic humoral factor (THF), asparaginase, arginase, arginine deiminase, adenosine deaminase, peroxidase dismutase, endotoxinase, catalase, chymotrypsin, lipase, uricase, adenosine diphosphatase, tyrosinase, bilirubin oxidase, glucose oxidase, glucosidase, galactosidase, glucocerebrosidase, and glucuronidase.

In the transformed cell according to the present disclosure, the protein of interest may be coded for by an amino acid sequence selected from the group consisting of SEQ ID NOS: 9 to 19.

In the transformed cell according to the present disclosure, the cell may be Escherichia coli, Bacillus subtilis, Bacillus thuringiensis, Salmonella typhimurium, Serratia marcescens, Pseudomonas spp. yeast, insect cells, CHO (Chinese hamster ovary) cells, W138, BHK, COS-7, 293, HepG2, 3T3, RIN, MDCK cells, or plant cells.

According to a fourth embodiment, the present disclosure provides a method for expressing and purifying a protein of interest by using a CSQ tag, the method comprising the steps of:
A) constructing an expressing vector carrying a nucleic acid coding for a fusion protein wherein the fusion protein includes a CSQ tag and a protein of interest;
B) transducing the expression vector into a host cell to obtain a transformant;
C) expressing the fusion protein including the CSQ tag and the protein of interest in the transformant;
D) precipitating the fusion protein including the CSQ tag and the protein of interest from the transformant with the aid of calcium; and
E) separating the protein of interest from the fusion protein with a hydrolase.

In the method for expressing and purifying a protein of interest according to the present disclosure, the CSQ tag may be coded for by an amino acid sequence of SEQ ID NO: 1 or 2. The amino acid sequence of SEQ ID NO: 1 may be encoded by the nucleotide sequence of SEQ ID NO: 3 and the amino acid sequence of SEQ ID NO: 2 may be encoded by the nucleotide sequence of SEQ ID NO: 4.

In the method for expressing and purifying a protein of interest according to the present disclosure, the CSQ tag and the protein of interest may be fused to each other via a hydrolase-cleavable peptide composed of an amino acid sequence selected from the group consisting of SEQ ID NOS: 5 to 8.

In the method for expressing and purifying a protein of interest according to the present disclosure, the protein of interest is selected from the group consisting of a polymer protein, a glycoprotein, a cytokine, a growth factor, a blood factor, a vaccine, a hormone, an enzyme, and an antibody. For example, the protein of interest may be selected from the group consisting of interleukin-2, blood clotting factor VII, blood clotting factor VIII, blood clotting factor IX, an immunoglobulin, horseradish peroxidase (HRP), a cytokine, α-interferon, β-interferon, γ-interferon, colony stimulating factor (GM-CSF), human fibronectin extra domain B (EBD), bone morphogenetic protein (BMP), fibroblast growth factor (FGF), vascular endothelial growth factor (VEGF), nerve growth factor (NGF), epidermal growth factor (EGF), insulin-like growth factor (IGF), trans-forming growth factor-α and -β (TGF-α and -β), brain-derived neurotrophic factor (BDNF), platelet-derived growth factor (PDGF), placental growth factor (PlGF), hepatocyte growth factor (HGF), fibroblast growth factor 1 and 2 (FGF-1 and -2), keratinocyte growth factor (KGF), glucagon-like peptide-1 (GLP-1), exendin, somatostatin, LHRH (luteinizing hormone-releasing hormone), adrenocorticotropic hormone, growth hormone-releasing hormone, oxytocin, thymosin alpha-1, corticotropin-releasing factor, calcitonin, bivalirudin, vasopressin, phospholipase-activating protein (PLAP), insulin, tumor necrosis factor (TNF), follicle-stimulating hormone, thyroid-stimulating hormone, antidiuretic hormone, pigmentary hormone, parathyroid hormone, luteinizing hormone, calcitonin gene-related peptide (CGRP), enkephalin, somatomedin, erythropoietin, hypothalamic releasing factor, prolactin, chorionic gonadotropin, tissue plasminogen activator, growth hormone releasing peptide (GHPR), thymic humoral factor (THF), asparaginase, arginase, arginine deiminase, adenosine deaminase, peroxidase dismutase, endotoxinase, catalase, chymotrypsin, lipase, uricase, adenosine diphosphatase, tyrosinase, bilirubin oxidase, glucose oxidase, glucosidase, galactosidase, glucocerebrosidase, and glucuronidase.

In the method for expressing and purifying a protein of interest according to the present disclosure, the protein of interest may be coded for by an amino acid sequence selected from the group consisting of SEQ ID NOS: 9 to 19.

A better understanding of the present disclosure may be obtained via the following examples which are set forth to illustrate, but are not to be construed as illustrating the present disclosure.

### <EXAMPLES>

### EXAMPLE 1: Separation of EDB Using CSQ-Tag

### 1-1. Cloning of EDB into CSQ1TEV or CSQ1Thrombin vector

In order to clone EDB into CSQ1TEV or CSQ1Thrombin vector, the following oligonucleotides EDB-F1 and EDB-B1 were synthesized (Bioneer Corporation, Daejeon, Korea). For comparison, a fusion protein of a well-known His tag or GST tag and EDB was prepared.
5'-AATGGATCCGAGGTGCCCCAACTCACTGAC-3' (SEQ ID NO: 20)
5'-ATTCTCGAGTTACGTTTGTTGTGTCAGTGTAGTAGG-3' (SEQ ID NO: 21)

For use in amplification, EDB-F1 20 pmol, EDB-B1 20 pmol, PCR PreMix 4 µl (Elpisbio, Daejeon, Korea) and a template 10 µg were mixed and added with distilled water to form a final volume of 20 µl. The mixture was subjected to PCR (95°C for 5 min, 30 cycles of: 95°C, 30 sec; 42°C, 30 sec; and 72°C, 45 sec, and 72°C, 5 min), followed by purification (PCR purification kit, GeneAll, Seoul, Korea) to obtain EDB14 (SEQ ID NO: 9), EDB21 (SEQ ID NO: 10), and EDB26 (SEQ ID NO: 11) genes. In order to insert the EDB genes into a CSQ1TEV or CSQ1Thrombin vector, the CSQ1TEV or CSQ1Thrombin vector and the insert DNAs were treated with restriction enzymes. About 1 µg of the insert DNA was incubated overnight with BamHI (New England Biolabs (NEB, Ipswich) and XhoI (NEB, Ipswich), followed by purifying the DNA digests through a PCR purification kit. In addition, about 40 µg of the CSQ1TEV or CSQ1Thrombin vector was incubated for 3 hours with CIAP (Calf Intestinal Alkaline Phosphatase) (NEB, Ipswich), followed by purification with a PCR purification kit. The DNA insert was ligated to the CSQTEV or CSQ1Thrombin vector at room temperature for 3 hours in the presence of T4 DNA ligase (Bioneer Corporation, Daejeon, Korea) (FIG. 3).

Next, transformation was made of the DNA resulting from ligating the EDB insert to the CSQ1TEV or CSQ1Thrombin vector. After being thawed on ice, 100 µl of DH5a competent cells was mixed and reacted for 30 min with 2 µl of the ligate solution. Heat shock was performed at 42°C for 1 min on the reaction mixture which was then added with 200 µl of SOC medium and cultured at 37°C for 30 min before being spread on plates. Of the colonies thus formed on the plate, six colonies were randomly picked up. The inserts were identified by PCR and DNA electrophoresis. The cloned colonies were committed to sequencing in Bioneer Corporation.

For comparison, a fusion protein of His tag or GST tag and EDB was cloned into pBT7-N-His and pBT7-N-GST vector provided from Bioneer Corporation in the same manner as described above.

### 1-2. Purification of CSQ1TEV-EDB, CSQ1Thrombin-EDB, His-EDB, and GST-EDB

The DNAs sequenced in Bioneer Corporation were all transformed into BL21 cells which were then spread on agar plates containing ampicillin. Colonies grown on the agar plates were each inoculated into 5 ml of LB broth containing ampicillin (50 µg/ml) and cultured overnight at 37°C while stirring at 200 rpm. The cell culture was mixed at a ratio of 1:1 with glycerol to give a 1-ml stock and stored at -80°C in a deep-freezer. The stock was inoculated in an amount of 100 µl into 20 ml of LB broth containing ampicillin (50 µg/ml) and cultured overnight at 37°C while stirring at 200 rpm. The culture was transferred to 400 ml of LB broth containing ampicillin (50 µg/ml) and incubated until OD=0.7. Then, additional incubation was carried out overnight at 18°C in the presence of 1 mM isopropyl-β-D-thiogalatopyranoside (IPTG) while stirring at 200 rpm. After centrifugation at 4°C and 4000 rpm for 20 min, the supernatant was discarded and the cell pellet was suspended in a lysis buffer (20 mM Tris (pH 8.0), 300 mM NaCl, and 10 mM imidazole). The suspension was stored overnight at -80°C and then completely thawed, followed by adding a solution of 10 mg of PMSF (phenyl methane sulfonyl fluoride) in 1 ml of DMSO to 1 ml of the thawed E. coli. The E. coli was lysed using a sonicator, followed by centrifugation at 4°C and 13,000 rpm for 1 hour. The supernatant was incubated with 20 mM CaCl₂ at 4°C for 1 hour and then centrifuged at 5000 rpm for 30 min. After removal of the supernatant, the pellet thus formed was suspended in EDTA to obtain CSQ1TEV-EDB and CSQ1Thrombin-EDB proteins (FIG. 4).

His-EDB was expressed and purified as follows. The pBT7-N-His vector having EDB14 cloned thereinto was transformed into BL21 cells which were then spread on agar plates containing ampicillin. Colonies grown on the agar plates were each inoculated into 5 ml of LB broth containing ampicillin (50 µg/ml) and cultured overnight at 37°C while stirring at 220 rpm. The cell culture was mixed at a ratio of 1:1 with glycerol to give a 1-ml stock and stored at -80°C in a deep-freezer. The stock was inoculated in an amount of 100 µl into 20 ml of LB broth containing ampicillin (50 µg/ml) and cultured overnight at 37°C while stirring at 220 rpm. The culture was transferred to 400 ml of LB broth containing ampicillin (50 µg/ml) and incubated until OD=0.7. Then, additional incubation was carried out overnight at 37°C in the presence of 1 mM isopropyl-β-D-thiogalatopyranoside (IPTG) while stirring at 220 rpm. After centrifugation at 4°C and 4000 xg for 10 min, the supernatant was discarded and the cell pellet was suspended in a lysis buffer (50mM sodium phosphate(pH 8.0), 300mM NaCl and 10mM imidazole). The suspension was stored overnight at -80°C and then completely thawed, followed by adding a solution of 10 mg of PMSF (phenyl methane sulfonyl fluoride) in 1 ml of isopropanol to 1 ml of the thawed E. coli.

The E. coli was lysed using a sonicator, followed by centrifugation at 4°C and 13,000 rpm for 1 hour. The supernatant was applied to a Ni-NTA affinity resin (Elposbio, Daejeon, Korea) which was previously washed with distilled water and a lysis buffer. Then, the resin was washed with 600 ml of a washing buffer (50mM sodium phosphate (pH 8.0), 300mM NaCl, and 20mM imidazole), followed by elution with a butter (50mM sodium phosphate (pH 8.0), 300mM NaCl, and 250mM imidazole) to acquire N-terminal His-tag EDB14 protein (FIG. 4).

GST-EDB was expressed and purified as follows. The pBT7-N-GST vector having EDB14 cloned thereinto was transformed into BL21 cells which were then spread on agar plates containing ampicillin. Colonies grown on the agar plates were each inoculated into 5 ml of LB broth containing ampicillin (50 µg/ml) and cultured overnight at 37°C while stirring at 220 rpm. The cell culture was mixed at a ratio of 1:1 with glycerol to give a 1-ml stock and stored at -80°C in a deep-freezer. The stock was inoculated in an amount of 100 µl into 20 ml of LB broth containing ampicillin (50 µg/ml) and cultured overnight at 37°C while stirring at 220 rpm. The culture was transferred to 400 ml of LB broth containing ampicillin (50 µg/ml) and incubated until OD=0.7. Then, additional incubation was carried out overnight at 37°C in the presence of 1 mM isopropyl-β-D-thiogalatopyranoside (IPTG) while stirring at 220 rpm. After centrifugation at 4°C and 4000 xg for 10 min, the supernatant was discarded and the cell pellet was suspended in a lysis buffer (50mM Tris(pH 7.5), 150mM NaCl and 0.05% NP-40). The suspension was stored overnight at -80°C and then completely thawed, followed by adding a solution of 10 mg of PMSF (phenyl methane sulfonyl fluoride) in 1 ml of isopropanol to 1 ml of the thawed E. coli.

The E. coli was lysed using a sonicator, followed by centrifugation at 4°C and 13,000 rpm for 1 hour. The supernatant was applied to the GST. Bind Agarose Resin (Elposbio, Daejeon, Korea) which was previously washed with distilled water and a lysis buffer. Then, the resin was washed with 600 ml of a washing buffer (50mM Tris-Cl (pH 8.0), 150mM NaCl, and 0.1mM EDTA), followed by elution with a (50mM Tris-Cl(pH 8.0), 150mM NaCl, 0.1mM EDTA, and 10mM Reduced(Free) glutathione) to acquire N-terminal GST-tag EDB14 protein (FIG. 4).

As a result, it was observed that EDB14 protein cannot be detected in the supernatant when using His-tag or GST-tag, but can be solubilized and detected in the supernatant when using CSQ-tag.

### 1-3. EDB isolation using TEV protease

In order to isolate only EDB from CSQ1TEV-EDB protein, the recombinant protein was incubated overnight at 30°C with 5 µl of TEV protease (Genscript, USA). The resulting digests were run by SDS-PAGE (FIG. 5). As a result, it was observed that the CSQ-tag and the EDB fusion protein can be easily segregated using TEV protease after precipitation with calcium.

### EXAMPLE 2: Separation of EGF Using CSQ-Tag

### 2-1. Cloning of EGF into CSQ1TEV or CSQ1Thrombin Vector

In order to clone EDB into CSQ1TEV or CSQ1Thrombin vector, the following oligonucleotides EGF-F1 and EGF-B1 were synthesized (Bioneer Corporation, Daejeon, Korea)
5'-AATGGATCCAACTCTGATAGCGAATGCCCG-3' (SEQ ID NO: 22)
5'-ATTCTCGAGTTA ACGCAGTTCCCACCATTT-3' (SEQ ID NO: 23)

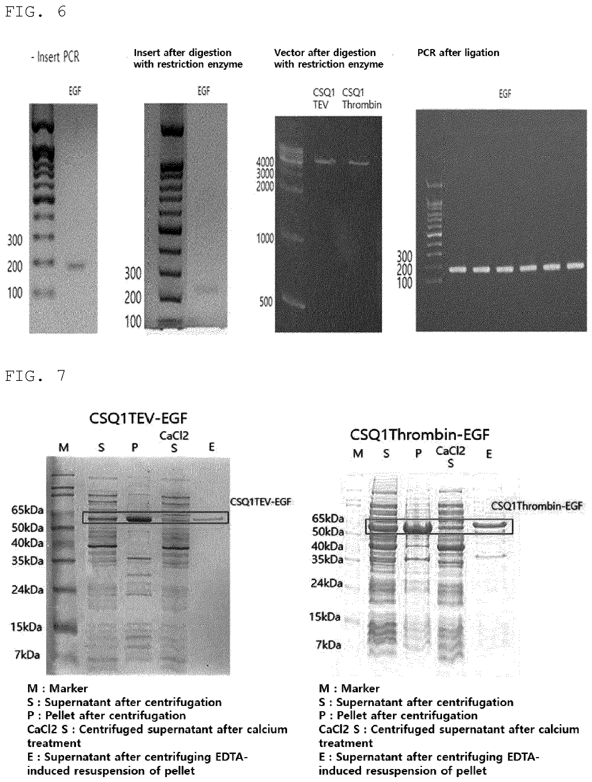

For use in amplification, EGF -F1 20 pmol, EGF-B1 20 pmol, PCR PreMix 4 µl (Elpisbio, Daejeon, Korea) and a template 10 µg were mixed and added with distilled water to form a final volume of 20 µl. The mixture was subjected to PCR (95°C for 5 min, 30 cycles of: 95°C, 30 sec; 42°C, 30 sec; and 72°C, 45 sec, and 72°C, 5 min), followed by purification (PCR purification kit, GeneAll, Seoul, Korea) to obtain a EGF gene (SEQ ID NO: 12). In order to insert the EGF gene into a CSQ1TEV or CSQ1Thrombin vector, the CSQ1TEV or CSQ1Thrombin vector and the insert DNAs were treated with restriction enzymes. About 1 µg of the insert DNA was incubated overnight with BamHI (New England Biolabs (NEB, Ipswich) and XhoI (NEB, Ipswich), followed by purifying the DNA digests through a PCR purification kit. In addition, about 40 µg of the CSQ1TEV or CSQ1Thrombin vector was incubated for 3 hours with CIAP (Calf Intestinal Alkaline Phosphatase) (NEB, Ipswich), followed by purification with a PCR purification kit. The DNA insert was ligated to the CSQTEV or CSQ1Thrombin vector at room temperature for 3 hours in the presence of T4 DNA ligase (Bioneer Corporation, Daejeon, Korea) (FIG. 6).

Next, transformation was made of the DNA resulting from ligating the EGF insert to the CSQ1TEV or CSQ1Thrombin vector. After being thawed on ice, 100 µl of DH5a competent cells was mixed and reacted for 30 min with 2 µl of the ligate solution. Heat shock was performed at 42°C for 1 min on the reaction mixture which was then added with 200 µl of SOC medium and cultured at 37°C for 30 min before being spread on plates. Of the colonies thus formed on the plates, six colonies were randomly picked up. The inserts were identified by PCR and DNA electrophoresis. The cloned colonies were committed to sequencing in Bioneer Corporation.

### 2-2. Purification of CSQ1Thrombin-EGF or CSQ1TEV-EGF

The DNA sequenced in Bioneer Corporation was transformed into BL21 cells which were then spread on agar plates containing ampicillin. Colonies grown on the agar plates were each inoculated into 5 ml of LB broth containing ampicillin (50 µg/ml) and cultured overnight at 37°C while stirring at 200 rpm. The cell culture was mixed at a ratio of 1:1 with glycerol to give a 1-ml stock and stored at -80°C in a deep-freezer. The stock was inoculated in an amount of 100 µl into 20 ml of LB broth containing ampicillin (50 µg/ml) and cultured overnight at 37°C while stirring at 200 rpm. The culture was transferred to 400 ml of LB broth containing ampicillin (50 µg/ml) and incubated until OD=0.7. Then, additional incubation was carried out overnight at 18°C in the presence of 1 mM isopropyl-β-D-thiogalatopyranoside (IPTG) while stirring at 200 rpm. After centrifugation at 4°C and 4000 rpm for 20 min, the supernatant was discarded and the cell pellet was suspended in a lysis buffer (20 mM Tris (pH 8.0), 300 mM NaCl, and 10 mM imidazole). The suspension was stored overnight at -80°C and then completely thawed, followed by adding a solution of 10 mg of PMSF (phenyl methane sulfonyl fluoride) in 1 ml of DMSO to 1 ml of the thawed E. coli. The E. coli was lysed using a sonicator before centrifugation at 4°C and 13,000 rpm for 1 hour. The supernatant was incubated with 20 mM CaCl₂ at 4°C for 1 hour and then centrifuged at 5000 rpm for 30 min. After removal of the supernatant, the pellet thus formed was suspended in EDTA to obtain CSQ1Thrombin-EGF and CSQ1TEV-EGF proteins (FIG. 7).

### 2-3. EGF isolation using thrombin protease

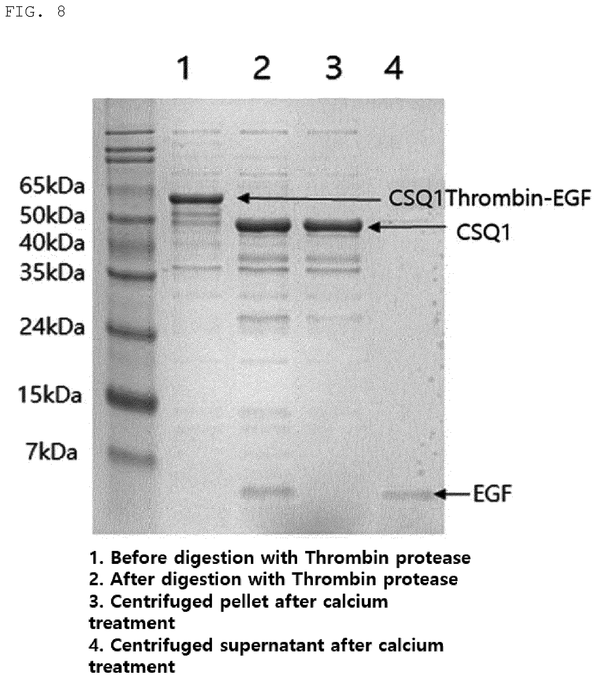

In order to isolate only EGF from CSQ1Thrombin-EGF protein, the recombinant protein was incubated overnight at 30°C with 5 µl of thrombin protease. The resulting digests were run by SDS-PAGE (FIG. 8). As a result, it was observed that the CSQ-tag and the EDB fusion protein can be easily segregated using thrombin protease.

### EXAMPLE 3: Separation of KGF1, VEGF, and FGF2 Using CSQ-Tag

### 3-1. Purification of CSQ1TEV-KGF1, CSQ1TEV-VEGF, and CSQ1TEV-FGF2

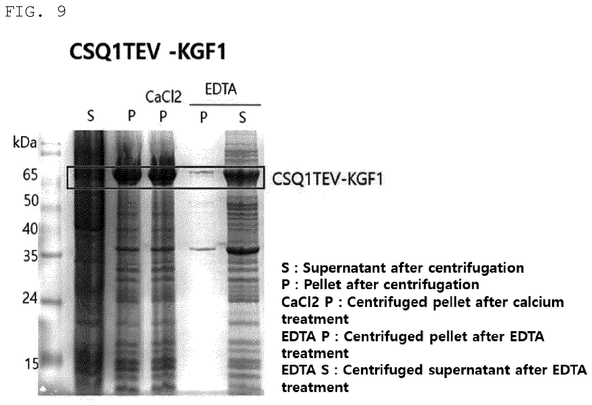

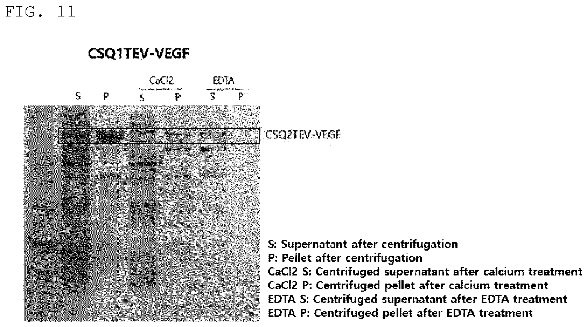

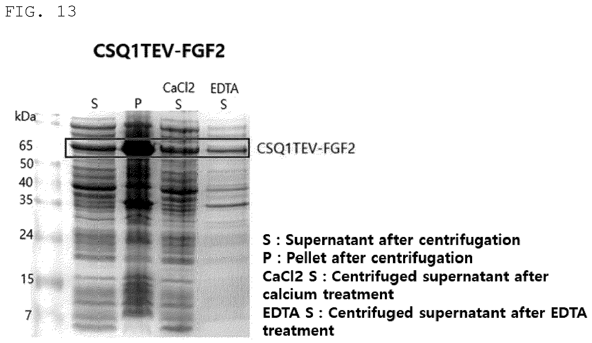

Genes of KGF1 (SEQ ID NO: 13), VEGF (SEQ ID NO: 14), and FGF2 (SEQ ID NO: 15) were synthesized in Bioneer Corporation and each cloned into CSQ1TEV which was then transformed into BL21 cells before being spread on agar plates containing ampicillin. Colonies grown on the agar plates were each inoculated into 5 ml of LB broth containing ampicillin (50 µg/ml) and cultured overnight at 37°C while stirring at 200 rpm. The cell culture was mixed at a ratio of 1:1 with glycerol to give a 1-ml stock and stored at -80°C in a deep-freezer. The stock was inoculated in an amount of 100 µl into 30 ml of LB broth containing ampicillin (50 µg/ml) and cultured overnight at 37°C while stirring at 200 rpm. The culture was transferred to 1 L of LB broth containing ampicillin (50 µg/ml) and incubated until OD=0.6. Then, additional incubation was carried out overnight at 17°C in the presence of 1 mM isopropyl-β-D-thiogalatopyranoside (IPTG) while stirring at 200 rpm. After centrifugation at 4°C and 4000 rpm for 20 min, the supernatant was discarded and the cell pellet was suspended in a lysis buffer (20 mM Tris (pH 8.0), 300 mM NaCl, and 10 mM imidazole). The suspension was stored overnight at -80°C and then completely thawed, followed by adding a solution of 10 mg of PMSF (phenyl methane sulfonyl fluoride) in 1 ml of DMSO to 1 ml of the thawed E. coli. The E. coli was lysed using a sonicator, followed by centrifugation at 4°C and 13,000 rpm for 1 hour. The supernatant was incubated with 20 mM CaCl₂ at 4°C for 1 hour and then centrifuged at 5000 rpm for 30 min. After removal of the supernatant, the pellet thus formed was suspended in EDTA to obtain CSQ1TEV-KGF1, CSQ1TEV-VEGF, and CSQ1TEV-FGF2 proteins (FIGS. 9, 11, and 13).

### 3-2. KGF1, VEGF, and FGF2 isolation using TEV protease

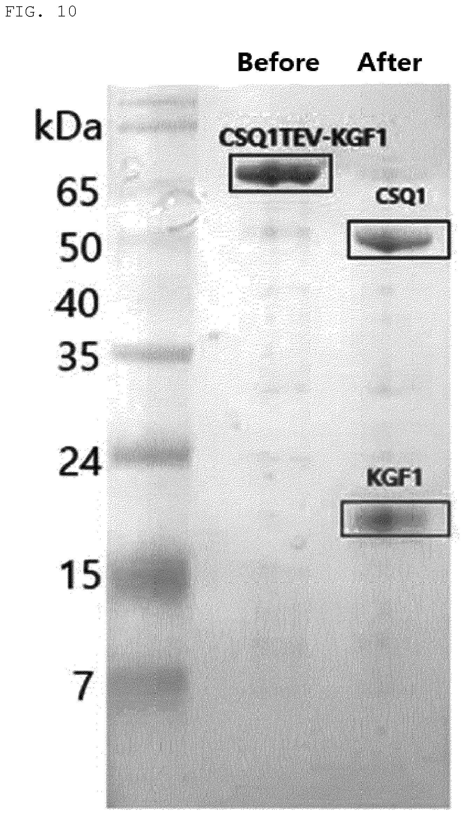

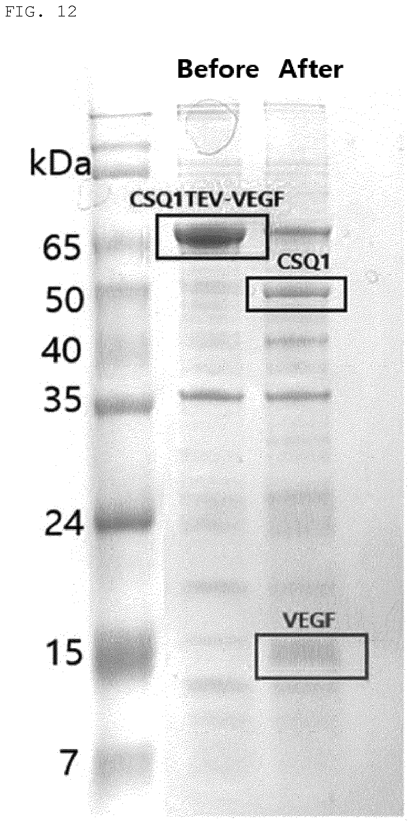

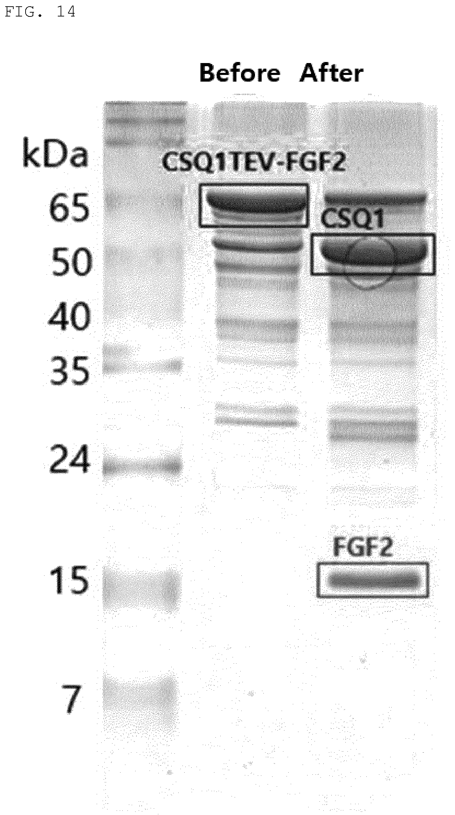

In order to isolate only KGF1 from CSQ1TEV-KGF1 protein (also from CSQ1TEV-VEGF and CSQ1TEV-FGF proteins), the recombinant protein was incubated overnight at 30°C with 5 µl of TEV protease (Genscript, USA). The resulting digests were run by SDS-PAGE (FIGS. 10, 12, and 14). As a result, it was observed that segregation was easily made between CSQ-tag and KGF1 fusion protein, between CSQ-tag and VEGF protein, and between CSQ-tag and FGF protein, using TEV protease.

### EXAMPLE 4: Separation of BMP2, TGFβ, HRP, and GLP1 Using CSQ-Tag

### 4-1. Cloning of BMP2 and TGFβ into TEVCSQ1 or ThrombinCSQ1 Vector

In order to clone BMP2 and TGFβ into TEVCSQ1 or ThrombinCSQ1 vector, the following oligonucleotides Amplify-Fl and Amplify-Bl were synthesized (Bioneer Corporation, Daejeon, Korea).
5'-CAGCAAGACAGCGATGGATCC-3' (SEQ ID NO: 24)
5'-CGACTTACAGGTGATCTCGAG-3' (SEQ ID NO: 25)

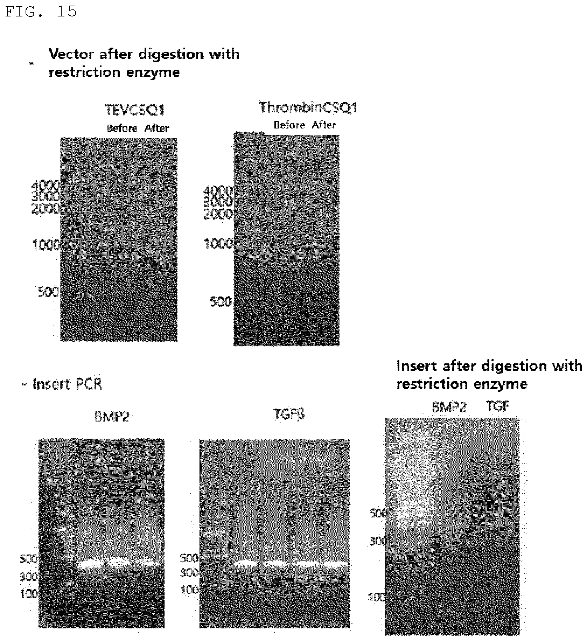

For use in amplification, Amplify-Fl 20 pmol, Amplify-Bl 20 pmol, PCR PreMix (Bioneer Corporation, Daejeon, Korea), and a template 10 µg were mixed and added with distilled water to form a final volume of 20 µl. The mixture was subjected to PCR (95°C for 5 min, 30 cycles of: 95°C, 30 sec; 47°C, 30 sec; and 72°C, 45 sec, and 72°C, 5 min), followed by purification (PCR purification kit, GeneAll, Seoul, Korea) to obtain BMP2 (SEQ ID NO: 16) and TGFβ (SEQ ID NO: 17) genes. In order to insert the BMP2 gene (or TGFβ gene) into a TEVCSQ1 or ThrombinCSQ1 vector, the CSQ1TEV or CSQ1Thrombin vector and the insert DNA were treated with restriction enzymes. About 1 µg of the insert DNA was incubated overnight with BamHI (New England Biolabs (NEB), Ipswich) and then for two days with XhoI (NEB, Ipswich). Thereafter, the DNA digests were purified using a PCR purification kit. In addition, about 3 µg of the TEVCSQ or ThrombinCSQ vector was incubated for 3 hours with CIAP (Calf Intestinal Alkaline Phosphatase) (Takara, Japan), followed by purification with a PCR purification kit. The DNA insert was ligated to the CSQTEV or CSQ1Thrombin vector at room temperature for 3 hours in the presence of T4 DNA ligase (Bioneer Corporation, Daejeon, Korea) (FIG. 15).

Next, transformation was made of the DNA resulting from ligating the BMP2 insert to the TEVCSQ1 or ThrombinCSQ1 vector. After being thawed on ice, 100 µl of DH5α competent cells was mixed and reacted for 30 min with 5 µl of the ligate solution. Heat shock was performed at 42°C for 30 sec on the reaction mixture which was then added with 200 µl of SOC medium and cultured at 37°C for 40 min before being spread on plates. The colonies thus formed on the plate were randomly picked up. The cloned colonies were committed to sequencing in Bioneer Corporation. Genes of HRP (SEQ ID NO: 18) and GLP1 (SEQ ID NO: 19) were also synthesized in Bioneer Corporation and cloned into TEVCSQ1 in the same manner as described above.

### 4-2. Purification of BMP2-TEVCSQ1 or BMP2-ThrombinCSQ1, TGFβ-TEVCSQ1 or TGFβ-ThrombinCSQ1, HRP-TEVCSQ1, and GLP1-TEVCSQ1

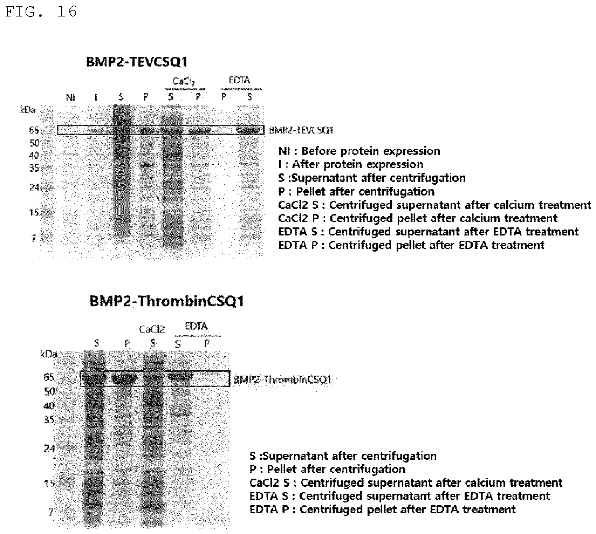

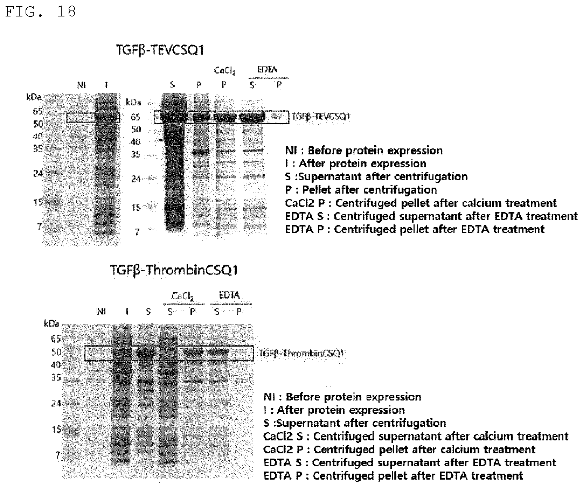

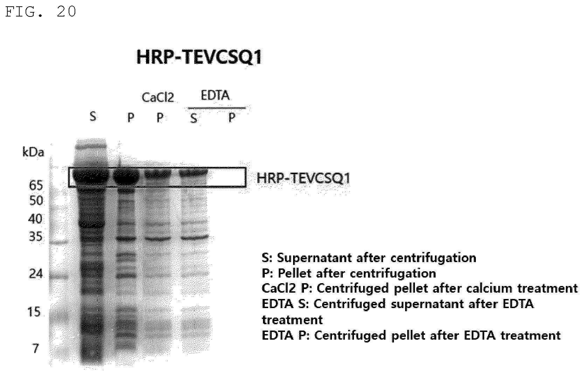

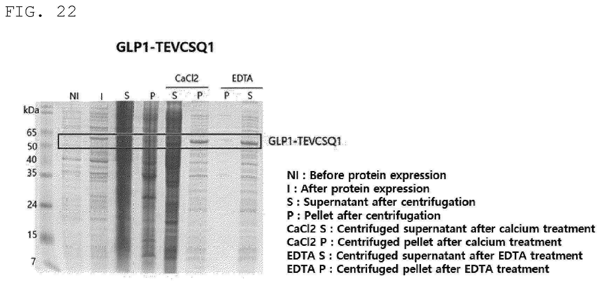

BMP2-TEVCSQ1, BMP2-ThrombinCSQ1, TGFβ-TEVCSQ1, TGFβ-ThrombinCSQ1, HRP-TEVCSQ1, GLP-TEVCSQ1 vectors sequenced in Bioneer Corporation were each transformed into BL21 cells which were then spread on agar plates containing ampicillin. Colonies grown on the agar plates were each inoculated into 5 ml of LB broth containing ampicillin (50 µg/ml) and cultured overnight at 37°C while stirring at 200 rpm. The cell culture was mixed at a ratio of 1:1 with glycerol to give a 1-ml stock and stored at -80°C in a deep-freezer. The stock was inoculated in an amount of 100 µl into 20 ml of LB broth containing ampicillin (50 µg/ml) and cultured overnight at 37°C while stirring at 200 rpm. The culture was transferred to 1 L of LB broth containing ampicillin (50 µg/ml) and incubated until OD=0.6. Then, additional incubation was carried out overnight at 17°C in the presence of 1 mM isopropyl-β-D-thiogalatopyranoside (IPTG) while stirring at 200 rpm. After centrifugation at 4°C and 4000 rpm for 20 min, the supernatant was discarded and the cell pellet was suspended in a lysis buffer (20 mM Tris (pH 8.0), 300 mM NaCl, and 10 mM imidazole). The suspension was stored overnight at -80°C and then completely thawed, followed by adding a solution of 10 mg of PMSF (phenyl methane sulfonyl fluoride) in 1 ml of DMSO to 1 ml of the thawed E. coli. The E. coli was lysed using a sonicator before centrifugation at 4°C and 13,000 rpm for 1 hour. The supernatant was incubated with 20 mM CaCl₂ at 4°C for 1 hour and then centrifuged at 5000 rpm for 30 min. After removal of the supernatant, the pellet thus formed was suspended in EDTA to obtain BMP2-TEVCSQ1 and BMP2-ThrombinCSQ1 proteins (FIGS. 16, 18, 20, and 22).

### 4-3. Isolation of BMP2, TGFβ, HRP, and GLP1 using TEV protease

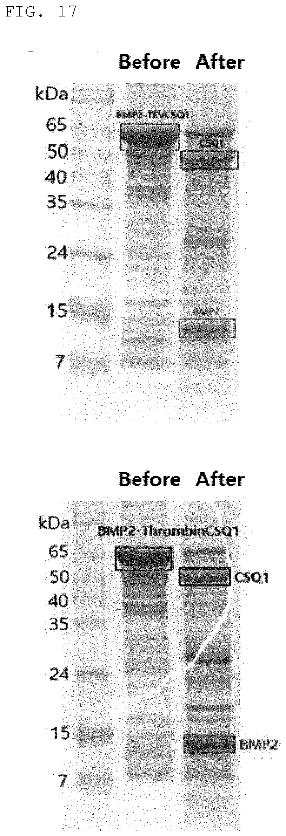

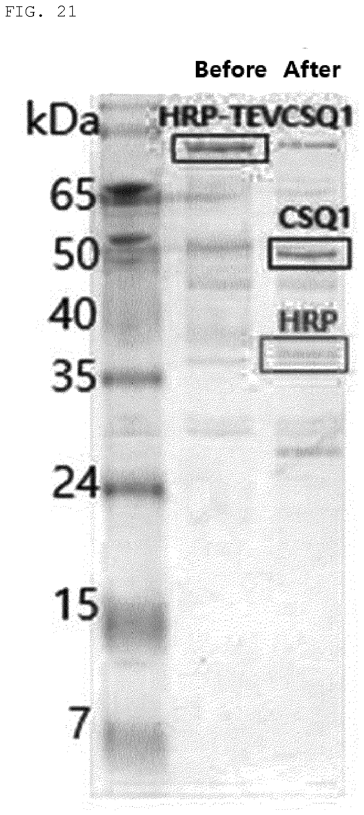

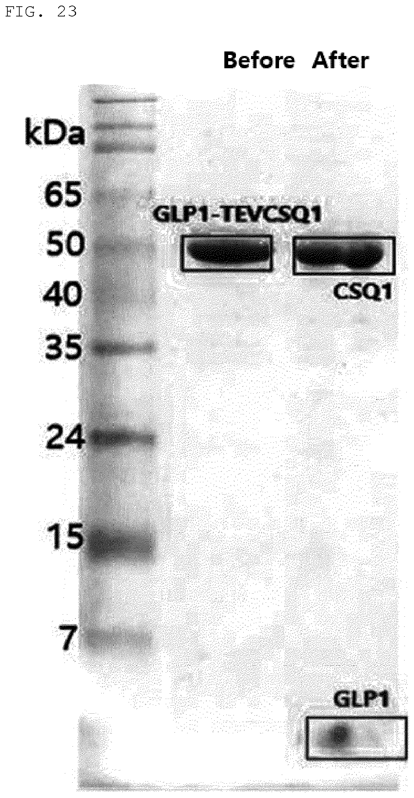

In order to isolate BMP2 from BMP2-TEVCSQ1 protein, the recombinant protein was incubated overnight at 30°C with 2 µl of TEV protease (Genscript, USA) (the same experiments were carried out for TGFβ-TEVCSQ1, HRP-TEVCSQ1, and GLP1-TEVCSQ1 proteins). The resulting digests were run by SDS-PAGE (FIGS. 17, 19, 21, and 23). As a result, it was observed that the CSQ-tag and the BMP2 fusion protein can be easily segregated using TEV protease.

### 4-4. Isolation of BMP2 and TGFβ using thrombin protease

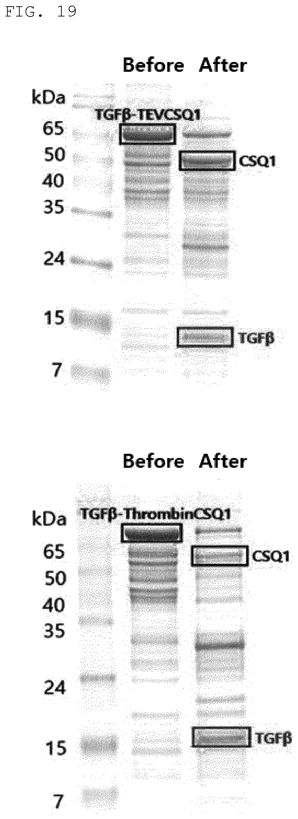

In order to isolate BMP2 from BMP2-ThrombinCSQ1 protein, the recombinant protein was incubated overnight at 22°C with 5 µl of thrombin protease (the same procedure was also carried out for TGFβ-TEVCSQ1). The resulting digests were run by SDS-PAGE (FIGS. 17 and 19). As a result, it was observed that the CSQ-tag and the BMP2 fusion protein can be easily segregated using thrombin protease.

### EXAMPLE 5: Separation of BMP2 and KGF1 Using CSQ-Tag

### 5-1. Cloning of BMP2 and KGF1 into CSQ2TEV or CSQ2Thrombin Vector

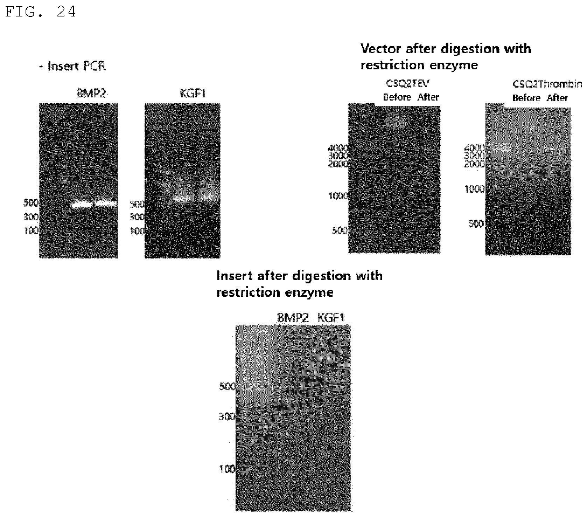

In order to clone BMP2 and KGF1 into CSQ2TEV or CSQ2Thrombin vector, Amplify-Fl and Amplify-Bl synthesized above (Bioneer Corporation, Daejeon, Korea) were employed. For use in amplification, Amplify-Fl 20 pmol, Amplify-Bl 20 pmol, PCR PreMix (Bioneer Corporation, Daejeon, Korea), and a template 10 µg were mixed and added with distilled water to form a final volume of 20 µl. The mixture was subjected to PCR (95°C for 5 min, 30 cycles of: 95°C, 30 sec; 47°C, 30 sec; and 72°C, 45 sec, and 72°C, 5 min), followed by purification (PCR purification kit, GeneAll, Seoul, Korea) to obtain BMP2 (SEQ ID NO: 16) and KGF1(SEQ ID NO: 13) genes. In order to insert the BMP2 gene (or KGF1 gene) into a CSQ2TEV or CSQ2Thrombin vector, the CSQ2TEV or CSQ2Thrombin vector and the insert DNA were treated with restriction enzymes. About 1 µg of the insert DNA was incubated overnight with BamHI (New England Biolabs (NEB), Ipswich) and then for two days with XhoI (NEB, Ipswich). Thereafter, the DNA digests were purified using a PCR purification kit. In addition, about 3 µg of the CSQ2TEV or CSQ2Thrombin vector was incubated for 3 hours with CIAP (Calf Intestinal Alkaline Phosphatase) (Takara, Japan), followed by purification with a PCR purification kit. The DNA insert was ligated to the CSQ2TEV or CSQ2Thrombin vector at room temperature for 3 hours in the presence of T4 DNA ligase (Bioneer Corporation, Daejeon, Korea) (FIG. 24).

Next, transformation was made of the DNA resulting from ligating the BMP2 insert to the CSQ2TEV or CSQ2Thrombin vector. After being thawed on ice, 100 µl of DH5α competent cells was mixed and reacted for 30 min with 5 µl of the ligate solution. Heat shock was performed at 42°C for 30 sec on the reaction mixture which was then added with 200 µl of SOC medium and cultured at 37°C for 40 min before being spread on plates. The colonies thus formed on the plate were randomly picked up. The cloned colonies were committed to sequencing in Bioneer Corporation.

### 5-2. Purification of CSQ2TEV-BMP2 or CSQ2Thrombin-BMP2, and CSQ2TEV-KGF1 or CSQ2Thrombin-KGF1

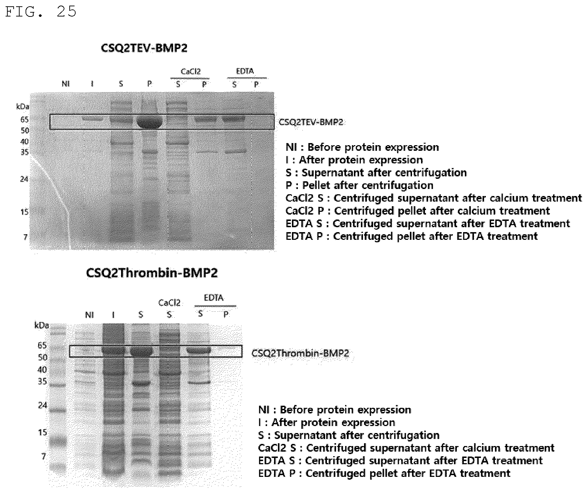

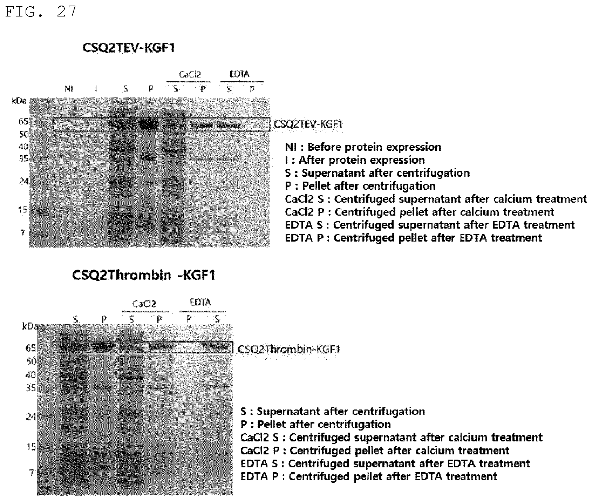

The DNAs sequenced in Bioneer Corporation were each transformed into BL21 cells which were then spread on agar plates containing ampicillin. Colonies grown on the agar plates were each inoculated into 5 ml of LB broth containing ampicillin (50 µg/ml) and cultured overnight at 37°C while stirring at 200 rpm. The cell culture was mixed at a ratio of 1:1 with glycerol to give a 1-ml stock and stored at -80°C in a deep-freezer. The stock was inoculated in an amount of 100 µl into 30 ml of LB broth containing ampicillin (50 µg/ml) and cultured overnight at 37°C while stirring at 200 rpm. The culture was transferred to 1 L of LB broth containing ampicillin (50 µg/ml) and incubated until OD=0.6. Then, additional incubation was carried out overnight at 17°C in the presence of 1 mM isopropyl-β-D-thiogalatopyranoside (IPTG) while stirring at 200 rpm. After centrifugation at 4°C and 4000 rpm for 20 min, the supernatant was discarded and the cell pellet was suspended in a lysis buffer (20 mM Tris (pH 8.0), 300 mM NaCl, and 10 mM imidazole). The suspension was stored overnight at -80°C and then completely thawed, followed by adding a solution of 10 mg of PMSF (phenyl methane sulfonyl fluoride) in 1 ml of DMSO to 1 ml of the thawed E. coli. The E. coli was lysed using a sonicator before centrifugation at 4°C and 13,000 rpm for 1 hour. The supernatant was incubated with 20 mM CaCl₂ at 4°C for 1 hour and then centrifuged at 5000 rpm for 30 min. After removal of the supernatant, the pellet thus formed was suspended in EDTA to obtain CSQ2TEV-BMP2 and CSQ2Thrombin-BMP2 proteins (FIGS. 25 and 27).

### 5-3. Isolation of BMP2 and KGF using TEV protease

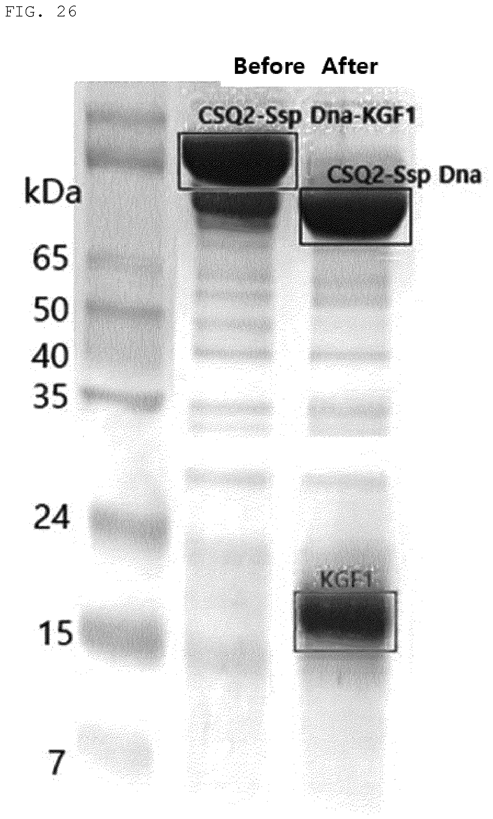

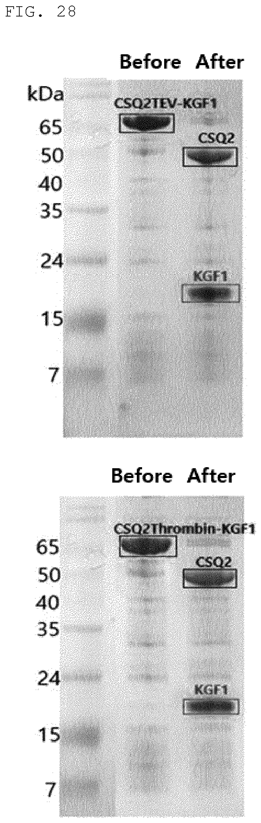

In order to isolate BMP2 from CSQ2TEV-BMP2 protein, the recombinant protein was incubated overnight at 30°C with 2 µl of TEV protease (Genscript, USA) (the same experiments were carried out for CSQ2TEV-KGF1 protein). The resulting digests were run by SDS-PAGE (FIGS. 26 and 28). As a result, it was observed that the CSQ-tag and the BMP2 fusion protein can be easily segregated using TEV protease.

### 5-4. Isolation of BMP2 and KGF using thrombin protease

In order to isolate BMP2 from CSQ2Thrombin-BMP2 protein, the recombinant protein was incubated overnight at 22°C with 5 µl of thrombin protease (the same procedure was also carried out for CSQ2Thrombin-KGF1 protein). The resulting digests were run by SDS-PAGE (FIGS. 26 and 28). As a result, it was observed that the CSQ-tag and the BMP2 fusion protein can be easily segregated using thrombin protease.

### EXAMPLE 6: Separation of HRP and GLP1 Using CSQ-Tag

### 6-1. Purification of HRP-TEVCSQ2 or HRP-ThrombinCSQ2 and GLP1-TEVCSQ2

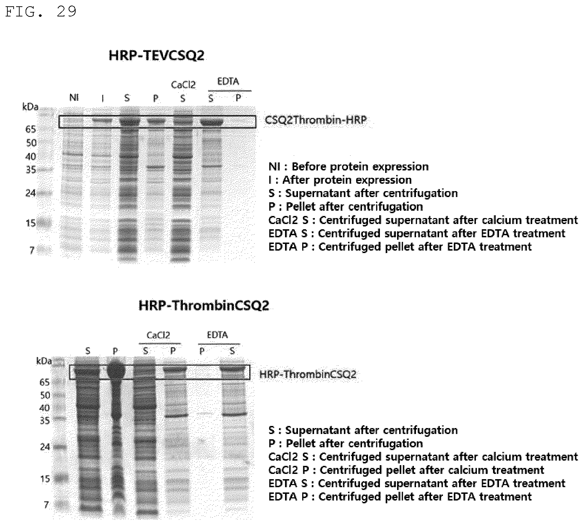

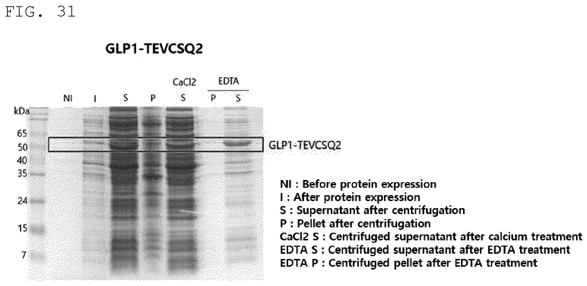

Genes of HRP (SEQ ID NO: 18) and GLP1 (SEQ ID NO: 19) sequenced in Bioneer Corporation were cloned into TEVCSQ2 and ThrombinCSQ2 vectors which were then transformed into BL21 cells, followed by spreading the cells on agar plates containing ampicillin. Colonies grown on the agar plates were each inoculated into 5 ml of LB broth containing ampicillin (50 µg/ml) and cultured overnight at 37°C while stirring at 200 rpm. The cell culture was mixed at a ratio of 1:1 with glycerol to give a 1-ml stock and stored at -80°C in a deep-freezer. The stock was inoculated in an amount of 100 µl into 30 ml of LB broth containing ampicillin (50 µg/ml) and cultured overnight at 37°C while stirring at 200 rpm. The culture was transferred to 1 L of LB broth containing ampicillin (50 µg/ml) and incubated until OD=0.6. Then, additional incubation was carried out overnight at 17°C in the presence of 1 mM isopropyl-β-D-thiogalatopyranoside (IPTG) while stirring at 200 rpm. After centrifugation at 4°C and 4000 rpm for 20 min, the supernatant was discarded and the cell pellet was suspended in a lysis buffer (20 mM Tris (pH 8.0), 300 mM NaCl, and 10 mM imidazole). The suspension was stored overnight at -80°C and then completely thawed, followed by adding a solution of 10 mg of PMSF (phenyl methane sulfonyl fluoride) in 1 ml of DMSO to 1 ml of the thawed E. coli. The E. coli was lysed using a sonicator before centrifugation at 4°C and 13,000 rpm for 1 hour. The supernatant was incubated with 20 mM CaCl₂ at 4°C for 1 hour and then centrifuged at 5000 rpm for 30 min. After removal of the supernatant, the pellet thus formed was suspended in EDTA to obtain HRP-TEVCSQ2, HRP-ThrombinCSQ2, and GLP1-TEVCSQ2 proteins (FIGS. 29 and 31) .

### 6-2. Isolation of HRP and GLP1 using TEV protease

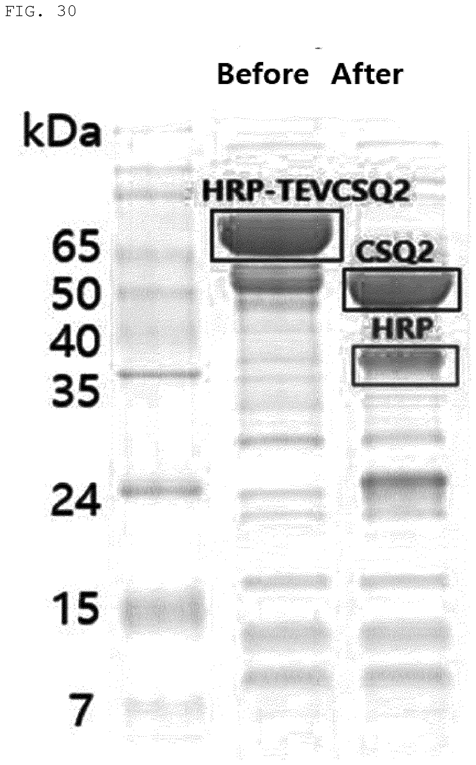

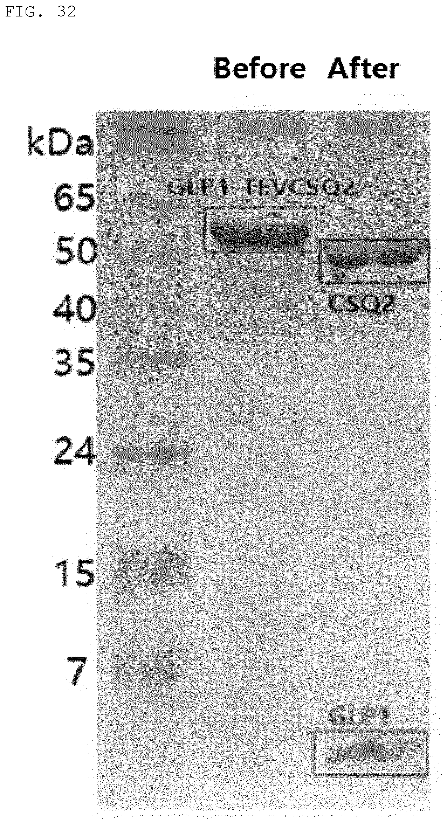

In order to isolate HRP from HRP-TEVCSQ2 protein, the recombinant protein was incubated overnight at 30°C with 2 µl of TEV protease (Genscript, USA) (the same experiments were carried out for GLP1-TEVCSQ2 protein). The resulting digests were run by SDS-PAGE (FIGS. 30 and 32). As a result, it was observed that the CSQ-tag and the GLP1 fusion protein can be easily segregated using TEV protease.

### EXAMPLE 7: Separation of EGF and KGF1 Using CSQ-Tag

### 7-1. Purification of CSQ1-Ssp Dna-EGF and CSQ2-Ssp Dna-KGF1

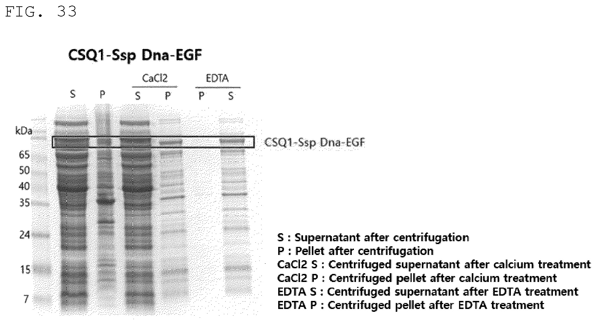

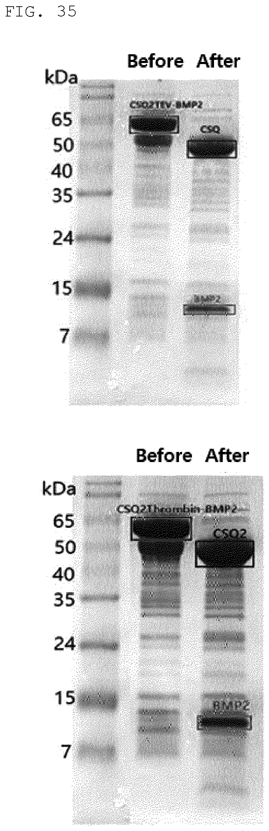

Genes of EGF (SEQ ID NO: 12) and KGF1(SEQ ID NO: 13) synthesized in Bioneer Corporation were cloned into CSQ1-Ssp Dna and CSQ2-Ssp Dna vectors which were then transformed into BL21 cells, followed by spreading the cells on agar plates containing ampicillin. Colonies grown on the agar plates were each inoculated into 5 ml of LB broth containing ampicillin (50 µg/ml) and cultured overnight at 37°C while stirring at 200 rpm. The cell culture was mixed at a ratio of 1:1 with glycerol to give a 1-ml stock and stored at -80°C in a deep-freezer. The stock was inoculated in an amount of 100 µl into 30 ml of LB broth containing ampicillin (50 µg/ml) and cultured overnight at 37°C while stirring at 200 rpm. The culture was transferred to 1 L of LB broth containing ampicillin (50 µg/ml) and incubated until OD=0.6. Then, additional incubation was carried out overnight at 17°C in the presence of 1 mM isopropyl-β-D-thiogalatopyranoside (IPTG) while stirring at 200 rpm. After centrifugation at 4°C and 4000 rpm for 20 min, the supernatant was discarded and the cell pellet was suspended in a lysis buffer (20 mM Tris (pH 8.0), 300 mM NaCl, and 10 mM imidazole). The suspension was stored overnight at -80°C and then completely thawed, followed by adding a solution of 10 mg of PMSF (phenyl methane sulfonyl fluoride) in 1 ml of DMSO to 1 ml of the thawed E. coli. The E. coli was lysed using a sonicator before centrifugation at 4°C and 13,000 rpm for 1 hour. The supernatant was incubated with 20 mM CaCl₂ at 4°C for 1 hour and then centrifuged at 5000 rpm for 30 min. After removal of the supernatant, the pellet thus formed was suspended in EDTA to obtain CSQ1-Ssp Dna-EGF and CSQ2-Ssp Dna-KGF1 proteins (FIGS. 33 and 35).

### 7-2. Isolation of EGF and KGF1 by pH

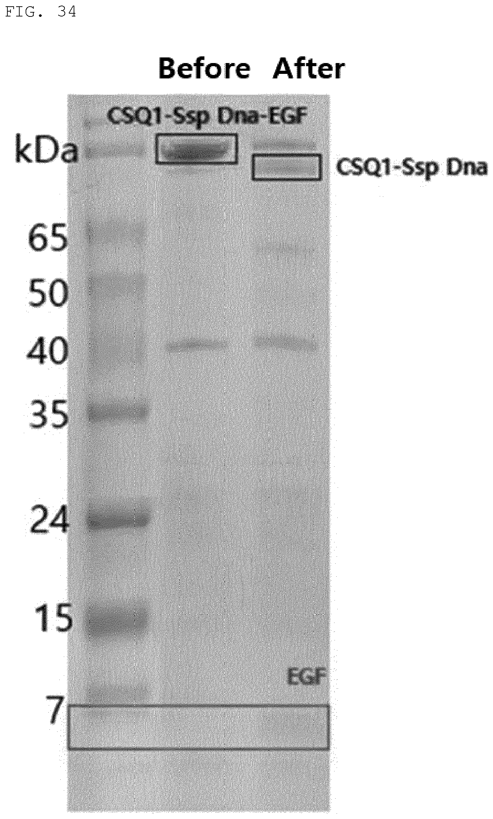

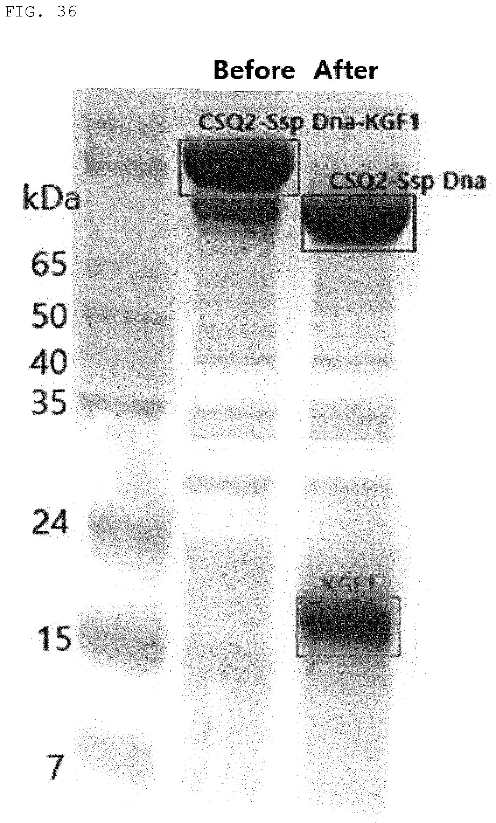

In order to isolate EGF from CSQ1-Ssp Dna-EGF protein, the recombinant protein was incubated overnight at room temperature with 20mM HEPES pH 6.5, 500mM NaCl buffer (the same experiments were carried out for CSQ2-Ssp Dna-KGF1 protein). The resulting reaction mixture was run by SDS-PAGE (FIGS. 34 and 36). As a result, it was observed that the CSQ-tag and the EGF fusion protein can be easily segregated by pH.

### EXAMPLE 8: Separation of EGF and BMP2 Using CSQ-Tag

### 8-1. Purification of EGF-GyrA-CSQ1 and BMP2-GyrA-CSQ2

Genes of EGF (SEQ ID NO: 12) and BMP2 (SEQ ID NO: 16) synthesized in Bioneer Corporation were cloned into GyrA-CSQ1 and GyrA-CSQ2 vectors which were then transformed into BL21 cells, followed by spreading the cells on agar plates containing ampicillin.

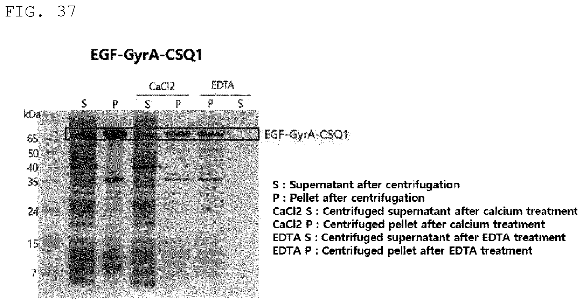

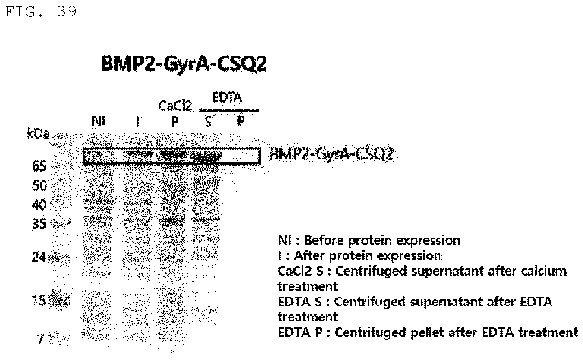

Colonies grown on the agar plates were each inoculated into 5 ml of LB broth containing ampicillin (50 µg/ml) and cultured overnight at 37°C while stirring at 200 rpm. The cell culture was mixed at a ratio of 1:1 with glycerol to give a 1-ml stock and stored at -80°C in a deep-freezer. The stock was inoculated in an amount of 100 µl into 30 ml of LB broth containing ampicillin (50 µg/ml) and cultured overnight at 37°C while stirring at 200 rpm. The culture was transferred to 1 L of LB broth containing ampicillin (50 µg/ml) and incubated until OD=0.6. Then, additional incubation was carried out overnight at 17°C in the presence of 1 mM isopropyl-β-D-thiogalatopyranoside (IPTG) while stirring at 200 rpm. After centrifugation at 4°C and 4000 rpm for 20 min, the supernatant was discarded and the cell pellet was suspended in a lysis buffer (20 mM Tris (pH 8.0), 300 mM NaCl, and 10 mM imidazole). The suspension was stored overnight at -80°C and then completely thawed, followed by adding a solution of 10 mg of PMSF (phenyl methane sulfonyl fluoride) in 1 ml of DMSO to 1 ml of the thawed E. coli. The E. coli was lysed using a sonicator before centrifugation at 4°C and 13,000 rpm for 1 hour. The supernatant was incubated with 20 mM CaCl₂ at 4°C for 1 hour and then centrifuged at 5000 rpm for 30 min. After removal of the supernatant, the pellet thus formed was suspended in EDTA to obtain EGF-GyrA-CSQ1 and BMP2-GyrA-CSQ2 proteins (FIGS. 37 and 39).

### 8-2. Segregation of EGF and BMP2 using dithiothreitol (DTT)

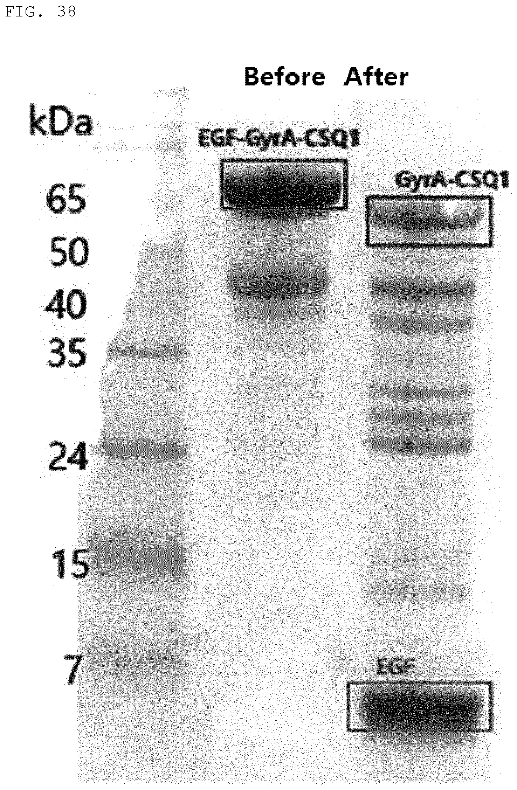

In order to isolate EGF from EGF-GyrA-CSQ1 protein, the recombinant protein was incubated overnight at room temperature with 20mM HEPES pH 8.5, 500mM NaCl, 40 mM DTT buffer (the same experiments were carried out for BMP2-GyrA-CSQ2 protein). The resulting reaction mixture was run by SDS-PAGE (FIGS. 38 and 36). As a result, it was observed that the CSQ-tag and the EGF fusion protein can be easily segregated using DTT.

Although the preferred embodiments of the present disclosure have been disclosed for illustrative purposes, those skilled in the art will appreciate that various modifications, additions and substitutions are possible, without departing from the scope and spirit of the present disclosure as disclosed in the accompanying claims.

## Claims

1. A fusion protein, comprising: a CSQ tag; and a protein of interest, wherein the CSQ tag acts to improve expression and water solubility of the protein of interest.

2. The fusion protein of claim 1, wherein the CSQ tag is coded for by an amino acid sequence of SEQ ID NO: 1 or 2.

3. The fusion protein of claim 1, wherein the CSQ tag is encoded by a nucleotide sequence of SEQ ID NO: 4 or 4.

4. The fusion protein of claim 1, wherein the CSQ tag and the protein of interest are fused to each other via a hydrolase-cleavable peptide composed of an amino acid sequence selected from the group consisting of SEQ ID NOS: 5 to 8.

5. The fusion protein of claim 1, wherein the protein of interest is selected from the group consisting of a polymer protein, a glycoprotein, a cytokine, a growth factor, a blood factor, a vaccine, a hormone, an enzyme, and an antibody.

6. The fusion protein of claim 5, wherein the protein of interest is selected from the group consisting of interleukin-2, blood clotting factor VII, blood clotting factor VIII, blood clotting factor IX, an immunoglobulin, horseradish peroxidase (HRP), a cytokine, α-interferon, β-interferon, γ-interferon, colony stimulating factor (GM-CSF), human fibronectin extra domain B (EBD), bone morphogenetic protein (BMP), fibroblast growth factor (FGF), vascular endothelial growth factor (VEGF), nerve growth factor (NGF), epidermal growth factor (EGF), insulin-like growth factor (IGF), trans-forming growth factor-α and -β (TGF-α and -β), brain-derived neurotrophic factor (BDNF), platelet-derived growth factor (PDGF), placental growth factor (PlGF), hepatocyte growth factor (HGF), fibroblast growth factor 1 and 2 (FGF-1 and -2), keratinocyte growth factor (KGF), glucagon-like peptide-1 (GLP-1), exendin, somatostatin, LHRH (luteinizing hormone-releasing hormone), adrenocorticotropic hormone, growth hormone-releasing hormone, oxytocin, thymosin alpha-1, corticotropin-releasing factor, calcitonin, bivalirudin, vasopressin, phospholipase-activating protein (PLAP), insulin, tumor necrosis factor (TNF), follicle-stimulating hormone, thyroid-stimulating hormone, antidiuretic hormone, pigmentary hormone, parathyroid hormone, luteinizing hormone, calcitonin gene-related peptide (CGRP), enkephalin, somatomedin, erythropoietin, hypothalamic releasing factor, prolactin, chorionic gonadotropin, tissue plasminogen activator, growth hormone releasing peptide (GHPR), thymic humoral factor (THF), asparaginase, arginase, arginine deiminase, adenosine deaminase, peroxidase dismutase, endotoxinase, catalase, chymotrypsin, lipase, uricase, adenosine diphosphatase, tyrosinase, bilirubin oxidase, glucose oxidase, glucosidase, galactosidase, glucocerebrosidase, and glucuronidase.

7. The fusion protein of claim 1, wherein the protein of interest is coded for by an amino acid sequence selected from the group consisting of SEQ ID NOS: 9 to 19.

8. A nucleic acid, including a nucleotide sequence coding for the fusion protein of any one of claims 1 to 7.

9. An expression vector, carrying the nucleic acid of claim 8.

10. A cell, transformed with the expression vector of claim 9.

11. The cell of claim 10, wherein the cell is Escherichia coli, Bacillus subtilis, Bacillus thuringiensis, Salmonella typhimurium, Serratia marcescens, Pseudomonas spp. yeast, insect cells, CHO (Chinese hamster ovary) cells, W138, BHK, COS-7, 293, HepG2, 3T3, RIN, MDCK cells, or plant cells.

12. A method for expressing and purifying a protein of interest by using a CSQ tag, the method comprising the steps of:
A) constructing an expressing vector carrying a nucleic acid coding for the fusion protein of any one of claims 1 to 7;
B) transducing the expression vector into a host cell to obtain a transformant;
C) expressing the fusion protein including the CSQ tag and the protein of interest in the transformant;
D) precipitating the fusion protein including the CSQ tag and the protein of interest from the transformant with aid of calcium; and
E) separating the protein of interest from the fusion protein with a hydrolase.
